(19) **Europäisches Patentamt**
**European Patent Office**
**Office européen des brevets**

(11) **EP 3 100 026 B1**

(12) **EUROPÄISCHE PATENTSCHRIFT**

(45) Veröffentlichungstag und Bekanntmachung des Hinweises auf die Patenterteilung:
**10.05.2023 Patentblatt 2023/19**

(21) Anmeldenummer: **15711054.5**

(22) Anmeldetag: **30.01.2015**

(51) Internationale Patentklassifikation (IPC):
**G01N 21/51** (2006.01)    **G01N 15/06** (2006.01)
**C12M 1/34** (2006.01)    **G01N 21/59** (2006.01)

(52) Gemeinsame Patentklassifikation (CPC):
**G01N 21/82; C12M 27/16; C12M 41/36;**
**G01N 15/06; G01N 21/1717; G01N 21/51;**
G01N 21/5907; G01N 2015/0046; G01N 2015/0053;
G01N 2015/0693

(86) Internationale Anmeldenummer:
**PCT/EP2015/051911**

(87) Internationale Veröffentlichungsnummer:
**WO 2015/114083 (06.08.2015 Gazette 2015/31)**

(54) **VERFAHREN, VORRICHTUNG UND SYSTEM ZUR AUTOMATISIERTEN BESTIMMUNG OPTISCHER DICHTEN ODER DER VERÄNDERUNG OPTISCHER DICHTEN VON REAKTIONSGEMISCHEN IN GESCHÜTTELTEN REAKTOREN**

METHOD, DEVICE, AND SYSTEM FOR THE AUTOMATED DETERMINATION OF OPTICAL DENSITIES OR OF THE CHANGE IN OPTICAL DENSITIES OF REACTION MIXTURES IN SHAKEN REACTORS

PROCÉDÉ, DISPOSITIF ET SYSTÈME DE DÉTERMINATION AUTOMATISÉE DE LA DENSITÉ OPTIQUE OU DE LA MODIFICATION DE LA DENSITÉ OPTIQUE DE MÉLANGES RÉACTIONNELS DANS DES RÉACTEURS AGITÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2014 DE 102014001284**

(43) Veröffentlichungstag der Anmeldung:
**07.12.2016 Patentblatt 2016/49**

(73) Patentinhaber: **Aquila Biolabs GmbH**
**52499 Baesweiler (DE)**

(72) Erfinder:
• **HERZOG, Konrad**
**52134 Herzogenrath (DE)**
• **FRANK, David**
**52134 Herzogenrath (DE)**

(74) Vertreter: **Roth, Andy Stefan**
**Dr. Roth Patentanwaltskanzlei**
**Kaistrasse 5**
**40221 Düsseldorf (DE)**

(56) Entgegenhaltungen:
**DE-A1- 10 125 600    DE-A1-102004 017 039**
**US-A1- 2005 219 526**

• **M. SAMORSKI ET AL: "Quasi-continuous combined scattered light and fluorescence measurements: A novel measurement technique for shaken microtiter plates", BIOTECHNOLOGY AND BIOENGINEERING, Bd. 92, Nr. 1, 5. Oktober 2005 (2005-10-05), Seiten 61-68, XP55194633, ISSN: 0006-3592, DOI: 10.1002/bit.20573**
• **Gwendolyn Leialoha Rivera: "DEVELOPMENT AND CHARACTERIZATION OF A PARALLEL MICROBIOREACTOR SYSTEM", , 1. Dezember 2004 (2004-12-01), XP55194657, Gefunden im Internet: URL:http://scholarspace.manoa.hawaii.edu/bitstream/handle/10125/10394/uhm_ms_3930_r.pdf?sequence=1 [gefunden am 2015-06-09]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

**Technisches Gebiet**

Die Erfindung betrifft ein Verfahren, eine Vorrichtung und ein System zur automatisierten Bestimmung der optischen Dichte und/oder der Veränderung der optischen Dichte von Reaktionsgemischen in geschüttelten Reaktoren im laufenden Schüttelbetrieb, wobei aus den ermittelten optischen Dichten und/oder den Veränderungen der optischen Dichte auf die Konzentration mindestens einer Komponente des Reaktionsgemisches zurückgeschlossen werden kann.

Die Erfindung kann insbesondere zur automatisierten Bestimmung der optischen Dichte und/oder der Veränderung der optischen Dichte und zur automatisierten Errechnung weiterer Parameter und Eigenschaften der Reaktionsgemische von Prozessen der Kultivierung von Zellen und Organismen, von Prozessen enzymatischer, biochemischer und chemischer Reaktionen und von anderen Reaktionsgemische beinhaltenden Prozessen genutzt werden, wobei das Reaktionsgemisch kontinuierlich geschüttelt wird.

Direkt aus den auf Basis der Erfindung ermittelten Messwerten lassen sich die optische Dichte und/oder die Veränderung der optischen Dichte und/oder fluidmechanische Parameter und Eigenschaften des Reaktionsgemisches bestimmen. Durch geeignete mathematische Verfahren, Modelle und Algorithmen können aus diesen Daten parallel zur laufenden Prozessführung weitere Parameter und Eigenschaften des Reaktionsgemisches ermittelt werden, beispielsweise Wachstumsraten, Substrataffinitäten, Dissoziationskonstanten, Inhibitionskonstanten, katalytische Umsatzraten, Sauerstofftransferraten, Aktivierungskonstanten, Repressionskonstanten und Substrat- oder Produktkonzentrationen.

Die Erfindung soll in geschüttelten Reaktoren wie beispielsweise Schüttelkolben und T-Flasks mit Füllvolumina unter einem Liter zum Einsatz kommen, um den derzeit hohen Messaufwand für biotechnologische, pharmazeutische, chemische und biochemische Screening- und Optimierungs- und Prozessüberwachungsverfahren zu minimieren und um eine hohe Parallelisierung dieser Verfahren bei gleichzeitig geringem personellem Mess-, Analyse- und Auswertungsaufwand zu ermöglichen. Auch soll aufgrund der nichtinvasiven Messmethode der Erfindung das Kontaminationsrisiko der bei den derzeit überwiegend eingesetzten, auf Probenentnahme basierenden, Messverfahren auf nahe Null reduziert werden. In Abgrenzung zum Stand der Technik soll die Erfindung unter verschiedensten Einsatzbedingungen robuste Messungen an geschüttelten Reaktoren im laufenden Schüttelbetrieb erlauben. Dies ist von besonderer Wichtigkeit, da Unterbrechungen des Schüttelbetriebes (z.B. zum Zweck der Messung optischer Dichten) den Verlauf einer im Reaktionsgemisch ablaufenden Reaktion gravierend beeinflussen können, sodass Ergebnisse aus geschüttelten Prozessen in vielen Fällen nur noch schwer und stark fehlerbehaftet auf gerührte oder anderweitig durchmischte Prozesse übertragbar sind. Durch Einsatz der Erfindung soll dieses Problem gelöst werden.

**Stand der Technik**

Aus dem Stand der Technik sind verschiedene Vorrichtungen und Methoden zur Bestimmung der optischen Dichte von Reaktionsgemischen in geschüttelten Bioreaktoren bekannt. Allgemein bekannte Grundlage dieser Technologien ist die Streuung und/oder Transmission von Licht durch sich im Lichtweg befindende Materie. Die Streu- und Transmissionsintensitäten stehen in einem mathematisch modellierbaren Zusammenhang mit der Konzentration der sich im Lichtweg befindenden und mit dem Licht interagierenden Materie, sodass die Messung der Intensitäten von Streu- und Transmissionslicht Rückschlüsse auf Stoffkonzentrationen erlaubt. Vorrichtungen zur Implementierung dieser Basismethode bestehen üblicherweise aus mindestens einer Lichtquelle zur Einleitung von Licht in das zu untersuchende Volumen und mindestens einem Lichtsensor zur Erfassung des gestreuten und/oder transmittierten Lichtes. Dieser grundlegende Aufbau und die grundlegende Methode können modifiziert werden, um bessere Ergebnisse zu erzielen. Die nachfolgend genannten Veröffentlichungen zum Stand der Technik werden daher nur insoweit erläutert, als sie über die allgemein bekannten Methoden und Vorrichtungen zur Bestimmung der optischen Dichte von Reaktionsgemischen hinausgehen und sie verbessern.

In US 2009/0075248 A1 werden eine Methode und eine Vorrichtung zur optischen Bestimmung der Partikelkonzentration in einem Medium über einen weiten linearen Bereich beschrieben. Dabei werden mehrere Lichtquelle-Lichtsensor-Paare zur Messung von Streu- und/oder Transmissionssignalen verwendet, die wiederum über verschiedene Algorithmen so kombiniert werden können, dass zwischen algorithmisch modifiziertem Messsignal und der Partikelkonzentration im Medium über einen Bereich von bis zu drei Konzentrationsgrößenordnungen (z.B. 0,1 g/L bis 100 g/L) eine lineare Abhängigkeit besteht. Laut Patentschrift vereinfacht dies die optische Bestimmung der Partikelkonzentration in einem Medium, da der normalerweise nichtlineare Zusammenhang zwischen Partikelkonzentration und einem einzigen direkt gemessenen Signal in einen linearen Zusammenhang überführt wird. Es werden verschiedene Ausgestaltungen von Methode und Vorrichtung beschrieben, z.B. zur nichtinvasiven Messung der Biomassekonzentration in einem Fermenter durch dessen lichtdurchlässige Wand, zur nichtinvasiven Messung der Biomassekonzentration in ungeschüttelten Kolben durch deren lichtdurchlässige Wand oder zur invasiven Messung mittels einer in das Medium versenkbaren Messsonde.

Nachteilig bezüglich Methode und Vorrichtung aus US 2009/0075248 A1 ist vor allem, dass sie nicht einsetzbar ist zur Messung an/in geschüttelten Systemen im laufenden Schüttelbetrieb. Die sich während des Schüttelns permanent ändernde Form und Verteilung des Mediums im geschüttelten Gefäß führt zu periodisch schwankenden Stärken des Transmissions- und Streusignals, welche durch die beschriebene Methode und Vorrichtung nicht berücksichtigt werden und somit zur Unbrauchbarkeit der Messung führen. Weiterhin nachteilig sind die beschriebenen Ausführungsformen, da sie sich nicht oder nur mit mangelhafter mechanischer Stabilität an einen geschüttelten Reaktor anbringen lassen.

Zudem ist bezüglich Methode und Vorrichtung aus US 2009/0075248 A1 zu bezweifeln, ob in einer realen Anwendung, insbesondere bei geschüttelten Systemen, die in der Patentschrift dargestellten Funktionsweisen, Messbereiche und Messgenauigkeiten tatsächlich erreichbar sind. Die in der Patentschrift angeführten Beispiele haben, zumindest für Anwendungen im Bereich der Kultivierung von Organismen, nur eine begrenzte Aussagekraft, da sie an Hefesuspensionen in wässriger 0,9% NaCl-Lösung durchgeführt wurden. Die in der Realität eingesetzten Kulturlösungen enthalten jedoch zwangsläufig organische Nahrungsquellen (z.B. Proteinlysate, Zellextrakte, Zucker, Aminosäuren, Lipide, etc.), welche ebenfalls zur Stärke des zu messenden Streu- und Transmissionssignals beitragen. Dies führt bei Messung der gleichen Zellkonzentration in verschiedener Medien zu einem unterschiedlichen Verhalten der beschriebenen Linearisierungsalgorithmen, da diese von Signalschwellwerten abhängig sind, deren Erreichen durch verschiedene Medien unterschiedlich stark beeinflusst wird. Die daraus erwachsenden Messungenauigkeiten sind ein großer Nachteil der Methode und Vorrichtung.

In US 8,405,033 B2 werden eine Methode und eine Vorrichtung zur optischen Bestimmung der Partikelkonzentration in einem Medium durch die Seitenwand eines Behältnisses beschrieben. Die Bestimmung der Partikelkonzentration erfolgt ausschließlich über das gestreute Licht, es wird kein Transmissionssignal gemessen. Um mit geringen Flüssigkeitsständen arbeiten zu können, wird eine Wellenlänge verwendet, die stark vom Medium absorbiert wird, sodass das gemessene Streusignal nur aus einem kleinen Volumen direkt vor Sensor und Lichtquelle stammt, welche zueinander in einem Abstand von mindestens 10% und maximal 1000% der mittleren Eindringtiefe des Lichts in das absorbierende Medium positioniert sind. Es werden verschiedene Ausgestaltungen von Methode und Vorrichtung beschrieben, z.B. zur nichtinvasiven Messung der Biomassekonzentration durch die Seitenwand oder den Boden eines Schüttelkolbens. Weiterhin wird in einem Satz die Aufnahme vieler Messpunkte pro Sekunde erwähnt, um Schwankungen des Flüssigkeitsstandes vor dem Detektor zu beobachten. Es wird jedoch nicht beschrieben, welcher messtechnische Zweck damit verfolgt werden soll.

Nachteilig bezüglich Methode und Vorrichtung aus US 8,405,033 B2 ist die Beschränkung auf Messungen des Streusignals, obwohl gerade bei niedrigen Partikelkonzentrationen (z.B. Zelldichten mit OD600 < 0,5) Genauigkeit und Zuverlässigkeit von Transmissionsmessungen deutlich besser sind als für Streulichtmessungen.

Nachteilig bezüglich Methode und Vorrichtung aus US 8,405,033 B2 ist auch deren Umsetzbarkeit in realen Geräten. So wird für das korrekte Funktionieren des durch die Firma BugLab LLC, 3350 Clayton Road, Suite 220, Concord, CA 94519 auf Grundlage dieses Patentes vermarkteten Geräts "OD Scanner" ein Flüssigkeitsstand von mindestens 3 cm vor Lichtquelle und Detektor benötigt. Dies lässt sich gerade bei der Verwendung kleiner Reaktionsgefäße (z.B. Schüttelkolben) nur durch eine Schräglage erreichen, wodurch die Messung nicht im laufenden Schüttelbetrieb erfolgen kann. Auch Messungen über ein im Patent vorgeschlagenes, unter dem Kolben angebrachtes System sind bei den häufig in der Industrie eingesetzten kleinen Volumina der Medien und Reaktionsgefäße (z.B. 20 mL in einem 200 mL Schüttelkolben) nicht praktisch realisierbar, da die erforderlichen Füllstände bei klassischen Kolbenbefüllungen von 10 % nicht erreicht werden können. Um auf der Grundlage dieses Patentes kontinuierliche Messungen im Schüttelbetrieb durchführen zu können, müsste daher entweder in sehr großen Medien-Volumina (z.B. 200 mL in einem 2000 mL Schüttelkolben) oder in deutlich überfüllten Schüttelkolben (Befüllung » 10 %) gemessen werden. Beide Varianten entbehren jeglichen Sinnes, da überfüllte Schüttelkolben sehr schlechte und vor allem nicht auf andere Reaktorsysteme skalierbare Mischungs- und Sauerstofftransferraten aufweisen und großvolumige Schüttelkolbenexperimente dem Grundsatz des minimalisierten Hochdurchsatz-Screenings widersprechen.

Zudem wurden auch die in US 8,405,033 B2 angeführten Beispiele an Hefesuspensionen in wässriger 0,9% NaCl-Lösung durchgeführt, und haben entsprechend der Argumentation bezüglich US 2009/0075248 A1 zumindest für Anwendungen im Bereich der Kultivierung von Organismen nur eine begrenzte Aussagekraft. Insbesondere im oben beschriebenen Messbereich geringer Partikelkonzentrationen kann die Streulichtmessung durch typischerweise optisch aktive Substanzen im Medium verfälscht werden. Die zur Korrektur des Fehlers benötigte Kalibration des Messgerätes auf jedes neu verwendete Medium stellt daher einen zusätzlichen Nachteil dar.

In JP 02/012217426 A wird eine Methode zur kontaktlosen, kontinuierlichen Messung des Wachstums einer Probe während der Kultivierung beschrieben. Funktionelles Grundprinzip der Methode ist die Transmissions- und Streulichtmessung an einer Position, an der durch die während des Schüttelns entstehende Flüssigkeitsverteilung nur eine sehr geringe Flüssigkeitsdicke vorliegt, sodass verwertbare Transmissionsmessungen durchgeführt werden können. Weiterhin wird die Möglichkeit der Messwertkorrektur über verschiedene Parameter beschrieben. Auch die automatisierte Zugabe und Entfernung von Kulturmedium mit Rückkopplung auf die Messwerte wird genannt. Ebenfalls beschrieben ist die Anpassung der Lichtquellen- und Detektorposition in Rückkopplung zu den Messwerten zur bestmöglichen Optimierung der Messung.

Nachteilig bezüglich der Methode aus JP 02/012217426 A ist die aus dem Patent hervorgehende geringe Breite des Messbereiches (maximal OD600 = 20), sodass bei höheren Zelldichten keine Messungen durchgeführt werden können. Im niedrigen Konzentrationsbereich (OD600 < 1) ist die Genauigkeit der Methode mangelhaft, wie aus den in der Patentschrift dargestellten Beispielen hervorgeht. Diese Nachteile werden auch durch das vom Patenteigentümer TAITEC Corp., 2693-1, Nishikata, Koshigaya City, Saitama, Japan hergestellte Produkt verdeutlicht, dessen Messbereich mit OD600-Werten zwischen 0,1 und 2,0 deutlich kleiner und damit von geringerem Nutzen ist als für die zuvor genannten Stand-der-Technik-Patente.

Ebenfalls von großem Nachteil bezüglich JP 02/012217426 A ist die starke Abhängigkeit der Messung von der Schüttelfrequenz (JP 02/012217426 A, Tabelle 2). Zur Anwendung der Methode bei unterschiedlichen Schüttelfrequenzen sind daher jeweils spezifische Korrektur- oder Kalibrierdaten notwendig, die aufzunehmen einen Nachteil in der praktischen Anwendung darstellt.

Nachteilig bezüglich der Methode aus JP 02/012217426 A ist auch ihre Anfälligkeit gegenüber Umgebungslicht. Die Messung an besonders dünnen Flüssigkeitslagen über Lichtquelle und Detektor kann zu einer Verfälschung der Messung durch umgebende Lichtquellen führen. Es wird im Patent zwar erwähnt, dass die Verwendung von infrarotem Licht diese Fehlerquelle eliminieren kann, allerdings erzeugen sowohl konventionelle Glühlampen als auch die beheizten Wände von thermoregulierten Inkubatoren nicht vernachlässigbare Mengen infraroter Strahlung, welche somit ebenfalls zu Messfehlern beitragen können.

Nachteilig bezüglich der Methode aus JP 02/012217426 A ist zudem die Art der Anordnung der Lichtsensoren und Lichtquellen, wie sie im Ausführungsbeispiel beschrieben und im entsprechenden Produkt ("OD-Monitor") der TAITEC Corp., 2693-1, Nishikata, Koshigaya City, Saitama, Japan umgesetzt wird. Die Messung parallel zur Schüttelebene hat, insbesondere aber nicht ausschließlich im Zusammenhang mit der im Patent beschriebenen Messung an dünnen Flüssigkeitsständen, den großen Nachteil, dass spezielle Schüttelfrequenzen, Reaktorgeometrien und Füllstände benötigt werden, um das zu analysierende Medium in den optischen Pfad zwischen Lichtquelle und Lichtsensor zu bringen. Dies limitierte nachteilig die universelle Einsetzbarkeit der Vorrichtung für verschiedene Reaktorgrößen, -geometrien und - füllstände. Zudem können prozessbedingte Änderungen der physikalischen und fluiddynamischen Parameter des geschüttelten Mediums dazu führen, dass sich die Dicke des sich im Lichtpfad befindlichen Flüssigkeitsstandes ändert, was zu signifikanten Messfehlern oder sogar zur vollständigen Unmessbarkeit (bei völliger Abwesenheit eines Flüssigkeitsfilms im optischen Pfad) führen kann. Beispiele für derartige Veränderungen des Mediums sind die Änderung der Viskosität infolge des Biomasseanstiegs, infolge von filamentösem Wachstum oder infolge der Sekretion gelbildender Substanzen sowie die Änderung des Medienvolumens infolge von Verdunstungseffekten.

Die Patente US 6,673,532 B2 und US 7,041,493 B2 beschreiben eine Methode und eine Vorrichtung zur optisch-chemischen Beobachtung von Bioprozessen. Unter anderem wird auch sehr allgemein die Möglichkeit zur Bestimmung der optischen Dichte der Kulturbrühe über Transmissionsmessungen beschrieben. Ebenso wird die Möglichkeit einer geschüttelten Prozessführung sowie die mit gemessenen Parametern rückgekoppelte Zugabe von Fluiden in die Kulturbrühe erwähnt.

Nachteilig bezüglich Methode und Vorrichtung aus US 6,673,532 B2 und US 7,041,493 B2 ist der invasive Charakter der Lichtleiter-basierten Messung der optischen Dichte, da durch ihn die große Gefahr der Kontamination der Kulturbrühe besteht. Zudem lässt sich diese Methode der Messung optischer Dichten nur sehr schwierig und mechanisch instabil in Reaktoren mit von idealer Zylinderform abweichender Geometrie (z.B. Schüttelkolben) implementieren. Auch der Verzicht auf streulichtbasierte Messungen der optischen Dichte stellt einen großen Nachteil dar, da Transmissionsmessungen nach der dort vorgestellten Methode bei höheren Biomassekonzentrationen (z.B. OD600 > 3) aufgrund der dann vorliegenden sehr geringen Transmissivität eine hohe Fehleranfälligkeit und Messungenauigkeit aufweisen. Bei noch höheren Biomassekonzentrationen, wie sie z.B. bei Hoch-Zelldichte-Fermentationen auftreten, sinkt die Transmissivität gegen Null, sodass hier ohne Streulichtanalysen keine verwertbaren Messergebnisse mehr erzielt werden können.

Von Nachteil bezüglich Methode und Vorrichtung aus US 6,673,532 B2 und US 7,041,493 B2 ist auch die Notwendigkeit der Nutzung eines speziell auf die optischen Vorrichtungen abgestimmten Schüttlers und Positioniertisches, da somit die Erweiterung bereits bestehender Schüttelgeräte um eine Analytik-Vorrichtung nicht möglich ist. Auch die in den Patenten dargestellte Kombination eines Positioniertisches mit einem Dispenser zur Zugabe von Fluiden in die Kulturbrühe ist nachteilig, da die Nutzung eines Dispensers für mehrere Reaktoren die Gefahr der Kreuzkontamination birgt. So können durch die Schüttelbewegung erzeugte Tröpfchen und Aerosole eines Reaktors während des Dispensiervorganges in Kontakt mit dem Dispenser gelangen, welcher diese dann im nächsten Dispensiervorgang an einen anderen Reaktor weitergibt und diesen somit möglicherweise mit fremden Zellen und Biomolekülen oder toxischen Medienbestandteilen kontaminiert.

In DE10 2004 017039 A1 werden Methode und Vorrichtung zur Erfassung von Prozessparametern von Reaktionsflüssigkeiten in mehreren geschüttelten Mikroreaktoren beschrieben. Teil der Beschreibung ist auch die Bestimmung der Biomassekonzentration über Streulichtmessungen.

Nachteilig bezüglich Methode und Vorrichtung aus DE 10 2004 017039 A1 ist der Verzicht auf Transmissionsmessungen, was wie bereits erwähnt zu einer schlechteren Genauigkeit von Messungen bei niedrigen Zelldichten (z.B. OD600 < 0,5) führt. Nachteilig ist auch die Limitierung der Methode und Vorrichtung auf Mikroreaktoren, obwohl ein großer Teil der biotechnologischen, biochemischen, mikrobiologischen, pharmazeutischen und chemischen Screeningprozesse in Volumina >1 mL durchgeführt wird.

Ebenfalls nachteilig bezüglich Methode und Vorrichtung aus DE 10 2004 017039 A1 ist das Konzept der Messung an einem geschüttelten Reaktor durch eine ortsfeste, nicht geschüttelte Lichtquelle/Detektor-Kombination. Die für diese Methode notwendige und im Patent beschriebene, auf die Schüttelbewegung abgestimmte gepulste Beleuchtung des Reaktors garantiert zwar das stete Auftreffen des Lichtes an derselben Stelle des Reaktors. Allerdings kann diese Methode nur dann korrekte Messdaten liefern, wenn sich zum Zeitpunkt jeder Messung auch stets derselbe Teil der schüttelbedingten Flüssigkeitsverteilung an der Messposition befindet. Durch die oben bereits beschriebenen prozessbegleitenden Änderungen des Flüssigkeitsvolumens und der Viskosität kann sich die schüttelbedingte Flüssigkeitsverteilung im Reaktor jedoch derart verändern, dass sich auch die entsprechende relative Messposition innerhalb dieser Flüssigkeitsverteilung verändert, sodass die Messergebnisse nicht mehr mit den früher im Prozess aufgenommenen Ergebnissen vergleichbar sind und eine korrekte Bestimmung der optischen Dichte unter den gegebenen fluidmechanischen Veränderungen nicht mehr erfolgen kann.

Sämtliche nach dem Stand der Technik bekannten Verfahren und Vorrichtungen zur Bestimmung der optischen Dichte und/oder der Veränderung der optischen Dichte von Reaktionsgemischen in geschüttelten Reaktoren beruhen auf einem grundlegenden messtechnischen Ansatz, bei dem versucht wird, durch bestimmte Maßnahmen die Beeinflussung einer Messung durch die schüttelbedingte Bewegung des Reaktionsgemisches im Reaktor zu minimieren oder zu eliminieren. Die zu diesem Zwecke eingesetzten Maßnahmen sind beispielsweise die Unterbrechung des Schüttelns (US 8,405,033 B2), die Wahl eines Lichtpfades, in dem die schüttelbedingte Bewegung des Reaktionsgemisches minimal ist (JP 02/012217426A), die Immersion von Lichtleitern zur Optimierung des Lichtpfades (US 6,673,532 B2 und US 7,041,493 B2) sowie der Einsatz von auf die Schüttelfrequenz abgestimmten Blitzlampen als Lichtquelle (DE 10 2004 017039 A1).

## Aufgabenstellung

Von diesem Stand der Technik ausgehend, ist es Aufgabe der Erfindung, ein Verfahren zur automatisierten Bestimmung der optischen Dichte und/oder der Veränderung der optischen Dichte von Reaktionsgemischen in geschüttelten Reaktoren im laufenden Schüttelbetrieb anzugeben, welche unter verschiedensten Prozessbedingungen zuverlässig arbeitet. Diese Zuverlässigkeit der Messung der optischen Dichte und/oder der Veränderung der optischen Dichte von Reaktionsgemischen in geschüttelten Reaktoren beinhaltet insbesondere aber nicht ausschließlich zuverlässiges Messen bei Umgebungslicht, zuverlässiges Messen bei unterschiedlichen Befüllungsständen des Reaktors, zuverlässiges Messen bei unterschiedlichen Schüttelfrequenzen, zuverlässiges Messen mit minimiertem Kalibrationsaufwand, zuverlässiges Messen in turbulenten Systemen und zuverlässiges Messen sowohl bei geringen als auch bei hohen optischen Dichten. Weiterhin soll das anzugebende Verfahren die automatische Bestimmung weiterer charakteristischer Parameter des Reaktionsgemisches oder der Reaktion ermöglichen und einen zuverlässigen und auf die Messergebnisse und Parameter abgestimmten Eingriff in das Reaktionsgeschehen erlauben. Zudem sollen eine Vorrichtung und ein System zur Durchführung des Verfahrens angegeben werden.

## Definitionen

In Bezug auf die nachfolgende Beschreibung der Erfindung und in Bezug auf die Patentansprüche werden folgende Definitionen getroffen.

Reaktoren sind Behältnisse, die insbesondere aber nicht ausschließlich zur Kultivierung von Organismen eingesetzt werden. Weitere Einsatzbereiche von Reaktoren sind unter anderem biokatalytische Prozesse unter Verwendung von Organismen und/oder Biomolekülen sowie andere chemische und/oder physikalische Prozesse, wobei als Prozesse insbesondere aber nicht ausschließlich alle Arten der Umwandlung, Auftrennung, Zusammenführung, Durchmischung, Größenänderung von insbesondere aber nicht ausschließlich chemischen Stoffen, Organismen, Partikeln, Lösungen, Emulsionen und Schäumen gelten. Als Reaktoren im Sinne der Erfindung gelten insbesondere aber nicht ausschließlich Schüttelkolben, T-Flasks, Mikrotiterplatten, Deep-Well-Plates, Schüttelfässer, Fermentation-Bags, Mehrzweckröhrchen und Zellkulturschalen.

Ein Reaktionsgemisch ist eine homogene oder heterogene Mischung aus mindestens zwei Komponenten, wobei sich diese in mindestens einer physikalischen, chemischen oder biologischen Eigenschaft unterscheiden. Als Komponenten gelten insbesondere aber nicht ausschließlich Organismen und Biomoleküle entsprechend der obigen Beschreibung, chemische Substanzen, Partikel, Lösungen, Emulsionen und Schäume.

Als Organismen gelten insbesondere aber nicht ausschließlich einzellige und mehrzellige prokaryote und eukaryote Lebewesen oder deren Lysate sowie natürliche oder synthetische Systeme aus grundlegenden Biomolekülen, wobei diese insbesondere aber nicht ausschließlich Nukleinsäuren, Proteine, Zucker und Lipide umfassen.

Die optische Dichte $E$ ist ein wellenlängenabhängiges Maß für die Veränderung der Intensität elektromagnetischer Strahlung beim Durchqueren eines Mediums und wird üblicherweise beschrieben als

$$E = log_{10}\left(\frac{I_0}{I}\right)$$

mit $I_0$ der Lichtintensität vor Durchquerung des Mediums und $I$ der Lichtintensität nach Durchquerung des Mediums. Demnach ergibt sich die Änderung der optischen Dichte $\Delta E$ zu:

$$\Delta E = E_2 - E_1 = log_{10}\left(\frac{I_1}{I_2}\right)$$

Die optische Dichte wird beeinflusst durch eine Vielzahl von Wechselwirkungen zwischen elektromagnetischer Strahlung und Medium, insbesondere aber nicht ausschließlich durch Absorption, Beugung, Streuung und Reflexion. Ist das durchquerte Medium ein Gemisch, so trägt jede Komponente des Gemisches mit komponentenabhängigem Anteil und komponentenabhängiger Art zur Wechselwirkung zwischen elektromagnetischer Strahlung und Medium und somit zur optischen Dichte bei. Infolge dieser Eigenschaften ist die optische Dichte ebenfalls ein Maß für die Konzentration der Komponenten im durchquerten Medium. Nach dem Gesetz von Lambert und Beer besteht in homogenen verdünnten Lösungen ein linearer Zusammenhang zwischen optischer Dichte und der Konzentration der transmissionsabschwächenden Komponenten.

Als Lichtsensor zählt jede Vorrichtung, die dazu geeignet ist, Licht aus dem Reaktor und/oder dem Reaktionsgemisch und/oder der Umgebung und/oder direkt von einer Lichtquelle zu detektieren, indem mindestens eine sich ändernde Eigenschaft des detektierten Lichtes (insbesondere die Intensität) eine elektrische Reaktion des Sensors (z.B. Änderung einer elektrischen Spannung, eines elektrischen Potentials, eines elektrischen Stroms) hervorruft, welche durch weitere elektronische Komponenten (z.B. Analog-Digital-Converter, Operationsverstärker, Komparatoren, Widerstände, Kondensatoren etc.) erkannt/ausgelesen und/oder weiterverarbeitet/umgewandelt/gespeichert werden kann. Ein Messpunkt ist somit ein direktes oder verarbeitetes/modifiziertes Abbild der elektrischen Reaktion des Sensors zu einer bestimmten Zeit. Als Lichtsensoren im Sinne der Erfindung gelten insbesondere aber nicht ausschließlich einzeln oder als Array vorliegende Fotodioden, Fotowiderstände und Fototransistoren, sowie 1D-CCD-Chips (Zeilensensor), 2D-CCD-Chips, 1D-CMOS-APS-Chips (Zeilensensor), 2D-CMOS-Chip sowie Lichtsensoren mit fluoreszierender Beschichtung (z.B. für UV-Detektion).

Als Lichtquelle zählt jede Vorrichtung, die dazu geeignet ist, Licht in den Reaktor und/oder das Reaktionsgemisch einzustrahlen. Als Lichtquellen im Sinne der Erfindung gelten insbesondere aber nicht ausschließlich LED, OLED, Laser, Glühlampen, Leuchtstoffröhren, Blitzröhren und Kombinationen dieser Lichtquellen mit mindestens einer fluoreszierenden Schicht.

Schütteln im Sinne der Erfindung ist die periodisch wiederholte Translation und/oder vollständige bzw. unvollständige Rotation des Reaktors entlang mindestens eines Translationspfades und/oder um mindestens eine Rotationsachse. Typische Schüttelarten sind insbesondere aber nicht ausschließlich das Orbitalschütteln (Translation des Reaktors entlang eines in sich geschlossenen, meist kreisförmigen, in der Schüttelebene liegenden, Translationspfades) und das Wippschütteln (unvollständige Rotation des Reaktors um mindestens eine, zum Gravitationskraftvektor nichtparallele, sondern meist orthogonale, Rotationsachse).

Als Rechner zählt jede elektronische Vorrichtung, die Daten (insbesondere arithmetische und logische) speichern und auf der Grundlage programmierbarer Vorschriften verarbeiten kann. Als Rechner im Sinne der Erfindung gelten insbesondere aber nicht ausschließlich Mikrocontroller, Mikroprozessoren, System-on-$\alpha$-Chip Rechner (SoC), PCs und Server.

Geeignete mathematische Verfahren und informationstechnische Algorithmen im Sinne der Erfindung sind all jene Verfahren und Algorithmen, die sinnvoll und nutzbringend zur Verarbeitung und/oder Auswertung erfindungsgemäß gemessener und/oder berechneter und/oder aus gemessenen Daten abgeleiteter und/oder anderweitig bestimmter oder erzeugter Daten eingesetzt werden können.

Eine Messreihe im Sinne der Erfindung ist eine aus mindestens zwei Messpunkten bestehende Sammlung von Messpunkten, wobei diese in einer im Vergleich zur Prozessgeschwindigkeit hinreichend kurzen Messzeit, in der die optische Dichte des Mediums messtechnisch als konstant angenommen werden kann, aufgenommen wurden. Sie kann zeitabhängig sein. Eine Messreihe kann sowohl aus Messpunkten eines einzelnen Lichtquelle-Lichtsensor-Paares als auch aus einer Kombination von Messpunkten verschiedener Lichtquelle-Lichtsensor-Paare bestehen.

Eine Zeitreihe im Sinne der Erfindung ist eine aus mindestens zwei Elementen bestehende, zeitlich geordnete Sammlung von zu unterschiedlichen Zeitpunkten erzeugten Elementen, wobei jedem Element die Zeit seiner Erzeugung zugeordnet ist. Elemente einer Zeitreihe können insbesondere aber nicht ausschließlich Messsignale/Messpunkte, Messreihen und Prozessparameter/-eigenschaften sein.

Die Aufgabenstellung wird gelöst durch ein Verfahren wie im Anspruch 1 und eine Vorrichtung wie im Anspruch 7.

In vorteilhafter Ausgestaltung der Erfindung Lichtquelle(n) und Lichtsensor(en) in einer Art und Weise positioniert und ausgerichtet sind, dass mindestens ein Lichtpfad nicht parallel zur Schüttelebene und/oder zur Schüttelachse verläuft.

Die Lösung der Aufgabe beruht, in Abgrenzung zum grundlegenden messtechnischen Ansatz des Stands der Technik, auf der Erkenntnis, dass die infolge des Schüttelvorganges periodisch schwankende Verteilung und/oder Form des Reaktionsgemisches im Reaktor genutzt werden kann, um zuverlässig die optische Dichte und/oder die Veränderung der optischen Dichte des Reaktionsgemisches zu bestimmen. Ausschlaggebend dabei ist die Aufzeichnung von mehreren Messpunkten (z.B. Streu- und Transmissionsintensitäten) in einer im Vergleich zur Prozessgeschwindigkeit (z.B. mikrobielle Wachstumsrate oder enzymatische Reaktionsgeschwindigkeit) kurzen Zeit. Das Ergebnis einer solchen Messreihe ist mindestens eine schüttelbedingt periodisch schwankende Kurve, deren Form durch die periodisch schwankenden Höhen des Reaktionsgemisches im jeweiligen Lichtpfad verursacht wird.

Die Verwendung einer im Vergleich zur Prozessgeschwindigkeit hinreichend kurzen Messzeit erlaubt die Annahme einer messtechnisch konstanten optischen Dichte in diesem Zeitraum, sodass im idealen, vollkommen ungestörten Fall die Messkurve eine von der optischen Dichte abhängige Funktion der Reaktionsgemischverteilung und/oder -form im Reaktor ist. Da Verteilung und/oder Form des Reaktionsgemisches im Reaktor infolge des periodischen Schüttelns Funktionen der Zeit sind, ist auch die ideale, vollkommen ungestörte Messkurve eine mathematisch modellierbare Funktion der Zeit.

Im Realfall wird jedes einzelne Messsignal nicht nur durch die optische Dichte und die Dicke des im Lichtpfad befindlichen Reaktionsgemisches beeinflusst, sondern auch durch unterschiedliche äußere Faktoren, insbesondere aber nicht ausschließlich durch Umgebungslicht und Inhomogenitäten im Reaktionsgemisch (z.B. Blasen, große Partikel). Diese Störfaktoren führen zu Ausreißern im idealen Kurvenverlauf einer Messreihe.

Durch Verwendung geeigneter mathematischer Verfahren, insbesondere aber nicht ausschließlich Verfahren der Statistik, der Regressionsanalytik, der Optimierung und der Ausgleichsrechnung, können durch äußere Faktoren hervorgerufene Ausreißer im Kurvenverlauf einer Messreihe erkannt werden, sodass aus einer Messreihe zuverlässig die optische Dichte und/oder die Veränderung der optischen Dichte des Reaktionsgemisches bestimmt werden kann.

Da, unter der Annahme einer messtechnisch konstanten optischen Dichte, der Kurvenverlauf einer Messreihe ein Abbild der Verteilung, Form und Bewegung des Reaktionsgemisches im Reaktor ist, kann jede Messreihe auch zur qualitativen Beurteilung und/oder quantitativen Bestimmung fluidmechanischer Eigenschaften und Parameter herangezogen werden. Dies ist von besonderer praktischer Relevanz sowohl zum tiefgreifenden Verständnis des untersuchten Prozesses als auch zur auf diesem Verständnis aufbauenden Skalierbarkeit des Prozesses (z.B. Up-Scaling mikrobieller Kultivierungen von kleinen Schüttelkolbenfermentationen auf große Rührkesselreaktorfermentationen).

Obwohl der Füllstand des Reaktors und die Schüttelfrequenz die Verteilung und/oder Form des Reaktionsgemisches im Reaktor beeinflussen, bleibt, bis auf wenige extreme und unrealistische Grenzfälle (z.B. vollständig gefüllter Reaktor), die prinzipielle Periodizität der schüttelbedingten Bewegung des Reaktionsgemisches im Reaktor erhalten. Infolgedessen erlaubt die Erfindung in einem sehr weiten Bereich von Füllständen und Schüttelfrequenzen die zuverlässige Bestimmung der optischen Dichte und/oder der Veränderung der optischen Dichte über geeignete mathematische Methoden der Auswertung der Messreihen. Ausschlaggebend für die Weite des Anwendungsbereiches der Erfindung ist die notwenige Existenz einer periodischen Schwankung der Verteilung und/oder Form des Reaktionsgemisches im Reaktor.

Die periodischen Schwankungen der Dicke des sich im Lichtpfad befindlichen Reaktionsgemisches können die zuverlässige Bestimmung der optischen Dichte und/oder der Veränderung der optischen Dichte allein über die Messung der Transmission erlauben. Zudem kann über geeignete mathematische Verfahren aus dem periodisch schwankenden Kurvenverlauf einer Messreihe mit hoher Zuverlässigkeit die optische Dichte und/oder die Veränderung der optischen Dichte auch in Bereichen bestimmt werden, die außerhalb der klassischen Auflösung handelsüblicher Spektrometer liegt (z.B. OD600 < 0,1).

Die zusätzliche Messung mindestens eines Streusignals kann zur Verifizierung der Transmissionsdaten genutzt werden und erweitert den Messbereich von Verfahren und Vorrichtung bis in Bereiche hoher optischer Dichten, in denen selbst aus den periodisch schwankenden Transmissionsmessreihen keine zuverlässige Bestimmung der optischen Dichte und/oder der Veränderung der optischen Dichte mehr möglich ist.

Die Messung mindestens eines Streu- und mindestens Transmissionssignals kann ebenfalls genutzt werden, um Schaumbildung auf geschüttelten flüssigen Reaktionsgemischen zu detektieren, da sich die Schaumbildung hauptsächlich auf das Transmissionssignal auswirkt und somit im Laufe der Schaumbildung die gemessenen Streuintensitäten nicht mehr zu den gemessenen Transmissionsintensitäten passen.

Mittels der durch die Erfindung ermöglichten Zuverlässigkeit der Messung optischer Dichten und/oder der Veränderung optischer Dichten kann auf viele der im Stand der Technik genannten Kalibrationsmessungen und Messwertkorrekturen zur Eliminierung von messwertbeeinflussenden Effekten wie z.B. Umgebungslicht, Schüttelfrequenz und Reaktorfüllstand verzichtet werden.

Über den Einsatz von mindestens zwei Lichtquelle-Lichtsensor-Lichtpfaden können, wenn benötigt, zu Beginn jedes Prozesses und auch im laufenden Prozess systeminterne Kalibrationen erfolgen.

Aus der aus mindestens einer Messreihe bestimmten optischen Dichte und/oder Veränderung der optischen Dichte kann über geeignete mathematische Verfahren direkt die Konzentration der optisch dichten Komponenten errechnet werden, insbesondere aber nicht ausschließlich die Konzentration von Zellen, Partikeln und gelösten, emulgierten oder eingeschäumten Stoffen.

In der Erfindung besteht zwischen Reaktor, Lichtquelle(n) und Lichtsensor(en) keine Relativbewegung während der Aufnahme einer Messreihe. In vorteilhafter Ausgestaltung der Erfindung ist die Messfrequenz um mindestens zwei Größenordnungen höher als die Schüttelfrequenz (z.B. Schüttelfrequenz von 3 Hz, Messfrequenz von 300 Hz). Dadurch können innerhalb einer einzigen Schüttelperiode hinreichend viele Messpunkte für eine Messreihe gesammelt werden, um eine robuste mathematische Auswertung der Messreihe zu erlauben. Entsprechend lassen sich über die hohen Messgeschwindigkeiten auch hohe zeitliche Dichten der aus den Messreihen bestimmten optischen Dichten und/oder Veränderungen der optischen Dichte umsetzen, die ein engmaschiges Monitoring des untersuchten Prozesses ermöglichen.

Die dadurch erreichte Datendichte erlaubt wiederum die zuverlässige und statistisch robuste Anwendung geeigneter mathematischer Verfahren zur Ableitung weiterer Prozessparameter aus den Zeitreihen der optischen Dichte und/oder der Veränderung der optischen Dichte. Derartige mathematische Verfahren umfassen insbesondere aber nicht ausschließlich Ausgleichsrechnungen ("Kurvenfitting") und Optimierungen von Modellfunktionen an die gemessenen Zeitreihen der optischen Dichte (z.B. mikrobielle Wachstumsmodelle, enzymkinetische Modelle).

Entsprechend der ausgewählten Modellfunktion können unterschiedliche Parameter bestimmt werden, insbesondere aber nicht ausschließlich Wachstumsraten, Substrataffinitäten, Dissoziationskonstanten, Inhibitionskonstanten, katalytische Umsatzraten, Sauerstofftransferraten, Aktivierungskonstanten, Repressionskonstanten und Substrat- oder Produktkonzentrationen.

Die Wahl der Modellfunktion kann durch den Nutzer oder im Rahmen des Verfahrens automatisch durch die Software erfolgen.

Sollten keine auf den zeitlichen Verlauf der optischen Dichte, der Veränderung der optischen Dichte, und/oder anderer Parameter und Eigenschaften des Reaktionsgemisches anpassbaren Modellfunktionen vorliegen, so können vorhandene Modellfunktionen mittels geeigneter mathematischer Verfahren und informationstechnischer Algorithmen automatisch modifiziert werden, um eine mathematische Modellierung der Parameter und/oder Eigenschaften zu erlauben. Zu diesem Zweck können Modellfunktionen mittels geeigneter mathematischer Verfahren und informationstechnischer Algorithmen auch automatisch neu generiert werden. Derartige Verfahren umfassen insbesondere aber nicht ausschließlich jegliche Art linearer und nichtlinearer Optimierungsverfahren sowie heuristische und gelenkte Suchalgorithmen, welche insbesondere aber nicht ausschließlich lineare Suchen, Suchbaum-Algorithmen, graphentheoretische Suchalgorithmen, optimierende Suchverfahren, direkte Suchverfahren, evolutionäre Algorithmen, neuronale Netzwerke und statistische Suchverfahren wie "Simulierte Abkühlung" oder Monte-Carlo-Algorithmen umfassen.

Die zur automatischen qualitativen Beurteilung und/oder quantitativen Bestimmung fluidmechanischer Eigenschaften aus den Messreihen einsetzbaren geeigneten mathematischen Verfahren und informationstechnischen Algorithmen umfassen unter anderem die vorgenannten Methoden.

In vorteilhafter Ausgestaltung der Erfindung sind sämtliche benötigten mathematischen Verfahren und informationstechnischen Algorithmen in einer zusammenhängenden Software und/oder in einzelnen Softwaremodulen implementiert.

In vorteilhafter Ausgestaltung der Erfindung ist die Vorrichtung modular aufgebaut, sodass mehrere Reaktoren und Reaktionsgemische über mindestens eine gemeinsame Recheneinheit und mindestens eine gemeinsam genutzte Software analysiert und ausgewertet werden können, wobei jedem Reaktor mindestens ein Lichtquelle-Lichtsensor-Paar zugeordnet ist.

In vorteilhafter Ausgestaltung der Erfindung ist der spektrale Bereich des in Reaktor und Reaktionsgemisch eingestrahlten Lichtes je nach Prozesserfordernis wählbar, um der wellenlängenabhängigen Interaktion des eingestrahlten Lichtes mit mindestens einer Komponente des Reaktionsgemisches Rechnung zu tragen. Vorteilhaft ist dies insbesondere aber nicht ausschließlich zur Optimierung des Streusignals an verschiedene Zelltypen und Zellgrößen in der Kultivierung von Organismen, da die Lichtstreuung von der Größe des streuenden Partikels abhängig ist. So streuen beispielsweise Bakterien optimal bei kürzeren Wellenlängen als Hefen oder humane Zellen. Die Auswahl eines spektralen Bereiches zur Einstrahlung in Reaktor und Reaktionsgemisch kann insbesondere aber nicht ausschließlich über die Verwendung von Lichtquellen mit eingeschränkten Spektralbereichen (z.B. LED, OLED, RGB-LED), oder über spektral breitbandige Lichtquellen (z.B. Glühlampe, Blitzlampe) in Kombination mit spektral selektiver Optik (insbesondere Filter, Prismen, Beugungsgitter) erfolgen.

In vorteilhafter Ausgestaltung der Erfindung kann auch der von mindestens einem Lichtsensor detektierte Spektralbereich wählbar sein, wobei insbesondere aber nicht ausschließlich Lichtsensoren mit besonderen spektralen Eigenschaften (z.B. sehr enger detektierbarer Wellenlängenbereich), oder spektral breitbandige Lichtsensoren in Kombination mit spektral selektiver Optik (insbesondere Filter, Prismen, Beugungsgitter) eingesetzt werden können.

Auf der Grundlage der unverarbeiteten Messreihen, der aus diesen bestimmten optischen Dichten und/oder Veränderungen der optischen Dichte, der aus den Messreihen bestimmten fluidmechanischen Parametern und der aus den Zeitreihen der optischen Dichten und/oder der Veränderung optischer Dichten und/oder der fluidmechanischen Parameter bestimmten weiteren Prozessparameter kann über eine auf die Messwerte und Parameter abgestimmte automatische Zugabe weiterer Komponenten zum Reaktionsgemisch automatisch in die Prozessführung eingegriffen werden, um z.B. bestimmte Parameter konstant zu halten oder deren Veränderung geplant zu beeinflussen. Beispiele für zuzugebende Komponenten sind insbesondere aber nicht ausschließlich reaktive Substanzen, Emulgatoren, Enzymlösungen, Zellsuspensionen, Substrate mikrobiellen Wachstums, Spurenelemente, Induktoren und Repressoren der Proteinexpression, Antibiotika, Wachstumsfaktoren, pH-Stellmittel, Antischaumsubstanzen und Lösungsmittel.

Für die automatisierte Zugabe von Komponenten zum Reaktionsgemisch können Dosiersysteme wie insbesondere aber nicht ausschließlich Pumpen oder schwerkraftgetriebene Systeme eingesetzt werden. In vorteilhafter Ausgestaltung der Erfindung sollen die Dosiersysteme die elektronisch regulierbare Zugabe variabler Mengen der zuzugebenden Komponente ermöglichen, wofür insbesondere aber nicht ausschließlich jegliche Art von Ventilen sowie ansteuerbare Pumpensysteme verwendet werden können. Die Regelung des Dosiersystems erfolgt durch eine Regelsoftware, welche in vorteilhafter Ausgestaltung der Erfindung in die Analyse-/Auswertungssoftware integriert ist.

In vorteilhafter Ausgestaltung der Erfindung befindet sich die Öffnung des Dosiersystems, über die die Zugabe von Komponenten in das Reaktionsgemisch erfolgt, über dem maximalen während des Schüttelns erreichbaren Stand des Reaktionsgemisches im Reaktor, sodass ein direkter Kontakt zwischen Dosiersystem und Reaktionsgemisch vermieden werden kann, um die Kontaminationsgefahr der Komponente im Reservoir des Dosiersystems zu begrenzen oder vollständig zu unterbinden.

In vorteilhafter Ausgestaltung der Erfindung ist jedem Reaktor ein eigenes Dosiersystem zugeordnet, um Kreuzkontaminationen auszuschließen und um parallel kontinuierliche Zugaben bei mehreren Reaktoren zu erlauben.

Eine weitere Möglichkeit der auf die aus den optischen Messungen ermittelten Parametern und Eigenschaften rückgekoppelten Prozessbeeinflussung ist die Temperierung von Reaktor und/oder Reaktionsgemisch. In vorteilhafter Ausgestaltung der Erfindung kommen dazu elektronisch regulierbare Temperiervorrichtungen zum Einsatz, insbesondere aber nicht ausschließlich Peltier-Elemente und flüssigkeitsgefüllte Kühl- und/oder Heizmäntel.

Anwendungsbereiche der Prozesssteuerung über die Regelung der Temperatur sind insbesondere aber nicht ausschließlich die Kultivierung von Organismen, die Produktion von Biomolekülen durch Organismen, enzymatisch oder anderweitig katalysierte Prozesse und nichtkatalysierte Prozesse mit prozessbegleitenden thermodynamischen Veränderungen. Die gezielte Temperierung erlaubt dabei die Beeinflussung von Parametern wie beispielsweise Wachstums- und/oder Reaktionsgeschwindigkeiten, Proteinexpressionsraten, Produktstabilität (insbesondere Proteinstabilität und Proteinfaltung), Löslichkeit, Aktivierungsenergie etc.

## Ausführungsbeispiel

Die Erfindung wird im Folgenden anhand der Abbildungen näher erläutert.

Abbildung 1 zeigt eine schematische Darstellung einer allgemeinen Ausführungsform des Verfahrens und der Vorrichtung.

Abbildung 2 zeigt exemplarisch für einen orbitalgeschüttelten Reaktor **1** die Abhängigkeit der gemessenen Lichtintensitäten von der Form und Verteilung des Reaktionsgemisches **2** im Reaktor **1.**

Abbildung 3 zeigt exemplarisch für einen wippgeschüttelten Reaktor **1** die Abhängigkeit der gemessenen Lichtintensitäten von der Form und Verteilung des Reaktionsgemisches **2** im Reaktor **1.**

Abbildung 4 zeigt eine Ausführungsform der Vorrichtung für orbitalgeschüttelte Schüttelkolben.

Abbildung 5 zeigt eine Ausführungsform der Vorrichtung für wipp- oder orbitalgeschüttelte T-Flaschen.

Abbildung 6 zeigt eine schematische Darstellung einer nichtmodularen Ausführung der Vorrichtung.

Abbildung 7 zeigt eine schematische Darstellung einer modularen Ausführung der Vorrichtung.

Abbildung 8 zeigt schematisch den Einsatz eines Sensorarrays als Lichtsensor **5.**

Abbildung 9 zeigt eine graphische Legende zur Erläuterung einiger wiederkehrender Bestandteile der Abbildungen 1 bis 8.

Die Abbildungen 10 bis 14 zeigen beispielhafte Mess- und Auswertungsergebnisse, die mittels des erfindungsgemäßen Verfahrens ermittelt wurden.

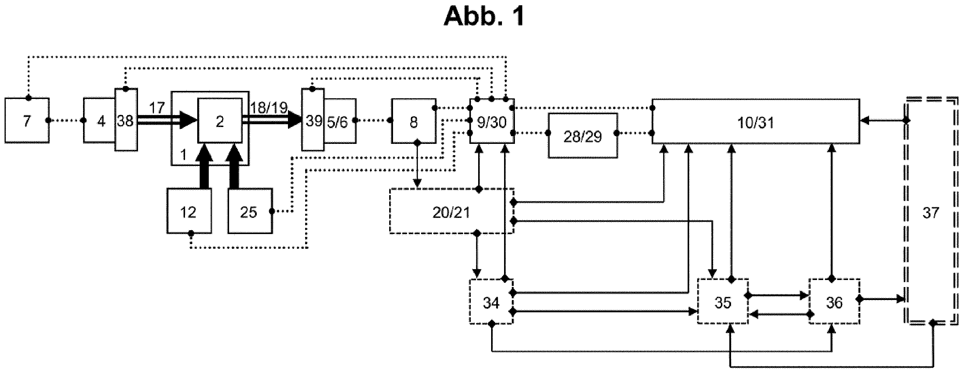

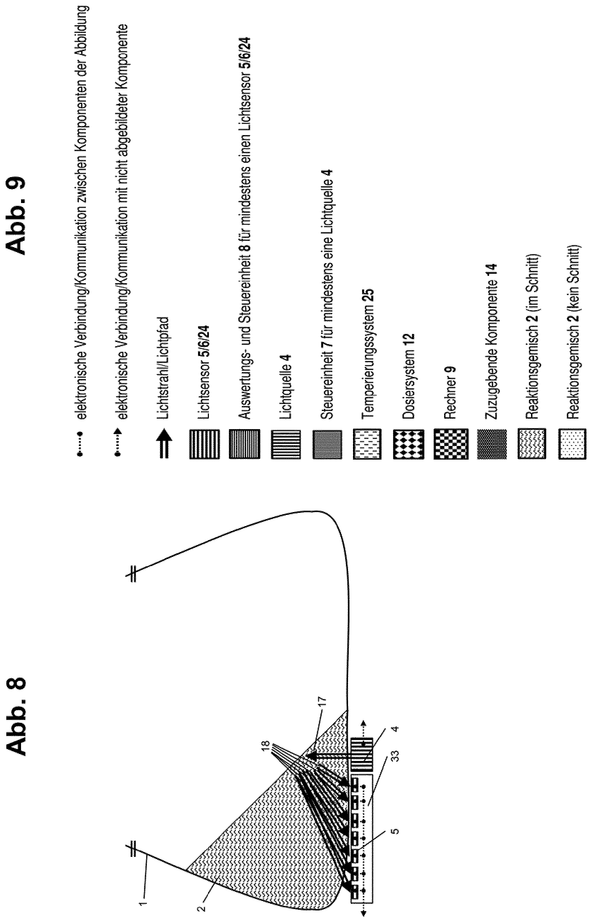

Gleiche oder gleich wirkende Elemente werden in den Abbildungen mit identischen Bezugszeichen versehen, wobei jeweils nur die Bezugszeichen verwendet werden, die zum Verständnis der Abbildung, auch im Kontext mit den anderen Abbildungen, notwendig sind. Auf Doppelbezeichnungen in gleichen oder ähnlichen Bestandteilen einer Abbildung wird daher weitestgehend verzichtet. Wiederkehrende Bestandteile der Abbildungen 1 bis 8 sind in Abbildung 9 zusammengefasst und werden daher nicht zusätzlich erläutert.

Abbildung 1 veranschaulicht die grundlegende Funktionsweise von Verfahren und Vorrichtung anhand eines schematischen Ausführungsbeispiels. In einem geschüttelten Reaktor **1** befindet sich das Reaktionsgemisch **2**. Von mindestens einer Lichtquelle **4** wird Licht **17** in den Reaktor **1** und das Reaktionsgemisch **2** eingestrahlt. Das eingestrahlte Licht **17** interagiert mit mindestens einer Komponente des Reaktionsgemisches **2** und verlässt dieses und den Reaktor **1** wieder, insbesondere aber nicht ausschließlich als rückgestreutes Licht **18** und/oder als vorwärtsgestreutes beziehungsweise transmittierendes Licht **19**. Das aus dem Reaktionsgemisch **2** austretende Licht **18/19** interagiert daraufhin mit mindestens einem Lichtsensor **5/6,** welcher die erfasste Lichtintensität in ein analoges elektrisches Signal umwandelt. Dieses wiederum wird von einer zu mindestens einem Lichtsensor **5/6** zugehörigen Auswertungs- und Steuereinheit **8** digitalisiert und als Messwert **20/21** gespeichert und/oder an mindestens einen Rechner **9** weitergegeben. Die auf Rechner **9** laufende Firmware **30** steuert dabei die Messwerterfassung, insbesondere aber nicht ausschließlich bezüglich der Messfrequenz, der Sensorsensitivität und der Verstärkung des analogen Messsignals. Zudem steuert die auf Rechner **9** laufende Firmware **30** über die Lichtquellen-Steuereinheit **7** die Strahlungsintensität der Lichtquelle **4.**

Sowohl Lichtquelle **4** als auch Lichtsensor **5/6** können mit einer Optik ausgestattet werden. Dabei modifiziert Optik **38** das von mindestens einer Lichtquelle **4** ausgestrahlte und in Reaktor **1** und Reaktionsgemisch **2** eingestrahlte Licht **17**, während Optik **39** das von Reaktor **1** und Reaktionsgemisch **2** ausgestrahlte Licht **18/19** modifiziert, welches daraufhin durch mindestens einen Lichtsensor **5/6** detektiert wird. Die für die Optiken **38** und **39** einsetzbaren Komponenten umfassen insbesondere aber nicht ausschließlich optische Filter, Linsen, Linsensysteme, Blenden, Blendensysteme, optische Spalte und Shutter, Polarisatoren, Lambdahalbe- und Lambdaviertelplatten, Beugungsgitter, Prismen und Lichtleiter. In einigen Ausführungsformen lassen sich die Optiken **38/39** von Rechner **9** mit Firmware **30** ansteuern.

Rechner **9** und Firmware **30** kommunizieren mit einem leistungsfähigeren Rechner **10** (z.B. PC) und der auf ihm laufenden Benutzersoftware **31** über mindestens ein Funkmodul **28** oder mindestens ein kabelgebundenes Kommunikationsmodul **29** oder aber direkt. Diese Kommunikation umfasst insbesondere aber nicht ausschließlich die Übertragung von Mess- und Analysedaten und anderen Informationen, wie z.B. Steuerbefehle für von Rechner **9** angesteuerte Bestandteile der Vorrichtung, Firmware- und/oder Softwareparameter, Zeit- und Taktsignale, Firmware-Updates und Lizenzinformationen.

Mehrere sequentiell in einem bestimmten Zeitintervall und mit einer bestimmten Messfrequenz erfassten Messdaten werden durch die Firmware **30** auf Rechner **9** und/oder die Benutzersoftware **31** auf Rechner **10** zu einer Messreihe **34** zusammengefasst. Die Verarbeitung einer Messreihe **34** mittels geeigneter mathematischer und informationstechnischer Verfahren und Algorithmen **36** liefert dann mindestens einen Parameter und/oder mindestens eine Eigenschaft **37** des im Reaktionsgemisch **2** ablaufenden Gesamtprozesses. Die Implementierung und Umsetzung geeigneter mathematischer und informationstechnischer Verfahren und Algorithmen **36** kann sowohl in Firmware **30** als auch in Benutzersoftware **31** erfolgen. In vorteilhafter Ausgestaltung der Erfindung und wie auch in Abbildung 1 dargestellt, wird der Großteil der eingesetzten Verfahren und Algorithmen **36** durch die Benutzersoftware **31** implementiert und ausgeführt, da die Rechenleistung von Rechner **10** üblicherweise deutlich höher und damit besser geeignet für komplexere Verfahren und Algorithmen **36** ist als die von Rechner **9.**

Zu verschiedenen Zeitpunkten ermittelte Messsignale **20/21,** Messreihen **34** und Prozessparameter/-eigenschaften **37** können durch Rechner **9** mit Firmware **30** und/oder Rechner **10** mit Benutzersoftware **31** zu mindestens einer Zeitreihe **35** der jeweiligen Datenart zusammengefasst werden. Die Verarbeitung einer Zeitreihe **35** mittels geeigneter mathematischer und informationstechnischer Verfahren und Algorithmen **36** liefert dann mindestens einen weiteren Parameter und/oder mindestens eine weitere Eigenschaft **37** des im Reaktionsgemisch **2** ablaufenden Gesamtprozesses.

Auf Grundlage mindestens einer Art der im laufenden Prozessgeschehen aus den optischen Messungen ermittelten Daten (Prozessparameter/-eigenschaften **37**, Zeitreihe **35**, Messreihen **34**, Messsignale **20/21**) kann im laufenden Prozess automatisiert in das Reaktionsgeschehen des Reaktionsgemisches **2** im Reaktor **1** eingegriffen werden. Dazu werden dem Reaktionsgemisch **2** über ein Dosiersystem **12** Substanzen zugegeben. Zudem kann über ein Temperierungssystem **25** die Temperatur von Reaktor **1** und Reaktionsgemisch **2** eingestellt werden, um so den laufenden Prozess gezielt zu beeinflussen.

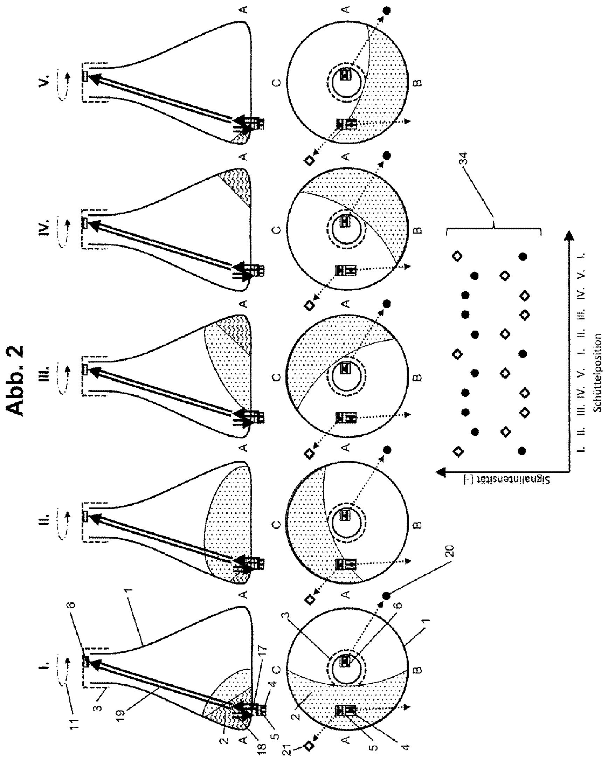

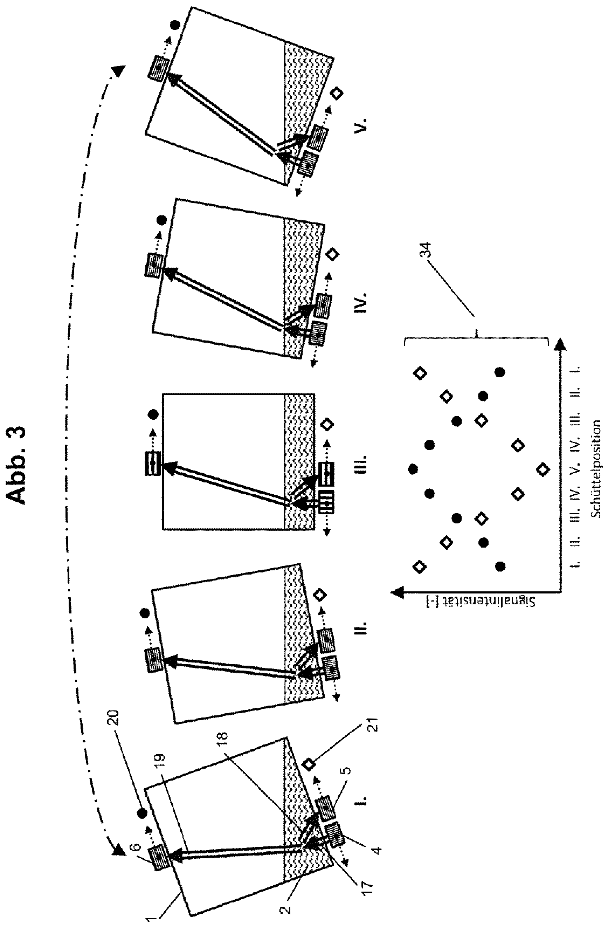

Abbildung 2 und Abbildung 3 veranschaulichen das Funktionsprinzip von Methode und Vorrichtung am Beispiel eines orbitalgeschüttelten Kolbens als Reaktor **1** (Abbildung 2) und am Beispiel eines wippgeschüttelten allgemeinen Reaktors **1** (Abbildung 3). In beiden dargestellten Ausführungsbeispielen befindet sich unterhalb des Reaktors **1** und des Reaktionsgemisches **2** eine Lichtquelle **4,** welche Licht **17** in den Reaktor **1** und in das Reaktionsgemisch **2** einstrahlt. Ebenfalls unterhalb des Reaktors **1** und des Reaktionsgemisches **2** befindet sich ein Lichtsensor **5** zur Detektion von rückgestreutem Licht **18**. Oberhalb des Reaktors **1** und des Reaktionsgemisches **2** ist ein Lichtsensor **6** zur Detektion von transmittiertem und vorwärts gestreutem Licht **19** platziert. Lichtsensor **5** liefert das Streusignal **21** und Lichtsensor **6** liefert das Transmissionssignal **20**.

Abbildung 2 und Abbildung 3 zeigen jeweils fünf exemplarische Schüttelpositionen (I. bis V.), wobei die Verteilung des Reaktionsgemisches **2** im Reaktor **1** schematisch zur Verdeutlichung des Funktionsprinzips der Erfindung gewählt ist. In beiden Abbildungen werden keine real aufgenommenen Messergebnisse dargestellt. Zur besseren Veranschaulichung der Verteilung des Reaktionsgemisches **2** im Reaktor **1** ist für den orbitalgeschüttelten Kolben in Abbildung 2 sowohl ein seitlicher Schnitt als auch eine Ansicht von unten dargestellt. Der seitliche Schnitt erfolgt, aus der Bildebene herausragend, entlang der Linie A-A der Ansicht von unten, sodass der Blick des Betrachters entlang der Linie B-C verläuft. Im Gegensatz dazu zeigt Abbildung 3 nur einen seitlichen Schnitt.

Im orbitalgeschüttelten Kolben in Abbildung 2 ergibt sich im Schüttelbetrieb eine charakteristische Verteilung des Reaktionsgemisches **2** im Reaktor **1,** wobei die Rotation des Reaktionsgemisches **2** im Reaktor **1** dazu führt, dass sich kontinuierlich periodisch die Höhe des Füllstandes über Lichtquelle **4** und Lichtsensor **5** und damit auch die Höhe des Füllstandes zwischen Lichtquelle **4** und Lichtsensor **6** ändert. Bei konstanter Wellenlänge und Intensität des eingestrahlten Lichts **17**, bei konstantem Außenlicht, unter der Annahme einer konstanten optischen Dichte des Reaktionsgemisches **2** und unter Abwesenheit von Inhomogenitäten wie Blasen oder Agglomeraten von Reaktionsgemisch-Komponenten, ist sowohl das Transmissionssignal **20** als auch das Streusignal **21** jeweils eine Funktion des verteilungsbedingten Füllstandes des Reaktionsgemisches **2** im Reaktor **1**. Ein Ausschnitt aus dem entsprechend periodischen Signalverlauf ist im Diagramm in Abbildung 2 schematisch dargestellt.

Ist der Füllstand über Lichtquelle **4** und Lichtsensor **5** maximal (Abbildung 2, Schüttelposition I.), so ist auch das Streusignal **21** von Lichtsensor **5** maximal, während das Transmissionssignal **20** von Lichtsensor **6** minimal ist, da hier im Vergleich zu den anderen Schüttelpositionen der geringste Teil des Lichtes durch das Reaktionsgemisch hindurch geht. Bei niedrigeren Füllständen über Lichtquelle **4** und Lichtsensor **5,** welche durch die Bewegung des Reaktionsgemisches **2** im Reaktor **1** bedingt sind, ist das Streusignal **21** von Lichtsensor **5** entsprechend niedriger und das Transmissionssignal **20** von Lichtsensor **6** entsprechend höher als bei Schüttelposition I.. Befindet sich kein Reaktionsgemisch über Lichtquelle **4** und Lichtsensor **5,** so ist das Streusignal **21** von Lichtsensor **5** minimal und das Transmissionssignal **20** von Lichtsensor **6** maximal (Abbildung 2, Schüttelpositionen III. und IV.).

Analog dazu ergeben sich in Abbildung 3 die Signalverläufe eines wippgeschüttelten Reaktors. Ist der Füllstand über Lichtquelle **4** und Lichtsensor **5** maximal (Abbildung 3, Schüttelposition I.), so ist auch das Streusignal **21** von Lichtsensor **5** maximal, während das Transmissionssignal **20** von Lichtsensor **6** minimal ist. Befindet sich kein oder kaum Reaktionsgemisch über Lichtquelle **4** und Lichtsensor **5,** so ist das Streusignal **21** von Lichtsensor **5** minimal und das Transmissionssignal **20** von Lichtsensor **6** maximal (Abbildung 3, Schüttelposition V.).

Sämtliche bei messtechnisch hinreichend konstanter optischer Dichte aufgenommenen Signale **20** und **21** werden zu Messreihen **34** zusammengefasst, welche entsprechend den Erläuterungen zu Abbildung 1 zur Ermittlung der optischen Dichte und/oder der Veränderung der optischen Dichte des Reaktionsgemisches **2** und vorzugsweise zur Ermittlung weiterer Prozessparameter und Prozesseigenschaften genutzt werden.

Infolge der Periodizität innerhalb einer Messreihe **34** können durch Störfaktoren verursachte Ausreißer im Kurvenverlauf effizient identifiziert und eliminiert werden, was die Zuverlässigkeit der Bestimmung der optischen Dichte und weiterer Prozessparameter und Prozesseigenschaften deutlich erhöht.

Aus der Form dieser Messreihen **34** lassen sich zudem qualitative und quantitative Rückschlüsse auf fluidmechanische Parameter und Eigenschaften des Reaktionsgemisches **2** ziehen, da die Form und Verteilung des Reaktionsgemisches **2** im Reaktor **1** und damit die Messsignale **20/21** innerhalb einer Messreihe **34** von den fluidmechanische Parametern und Eigenschaften des Reaktionsgemisches **2** abhängig sind. Ein prozesstechnisch bedeutsamer fluidmechanischer Parameter ist dabei die Viskosität des Reaktionsgemisches **2,** welche sich stark auf dessen Form und Verteilung im Reaktor **1** auswirken kann. Die Erfindung ermöglicht daher auf der Grundlage ihres Messverfahrens die Beurteilung der Viskosität des Reaktionsgemisches **2,** was von hohem Wert ist für die Überwachung und Optimierung verschiedenster biotechnologischer und chemischer Prozesse, bei denen sich im Laufe des Prozesses die Viskosität ändert (z.B. infolge von Zellwachstum, filamentösem Wachstum, Bildung von gelbildenden Substanzen, Bildung von Polymeren, etc.).

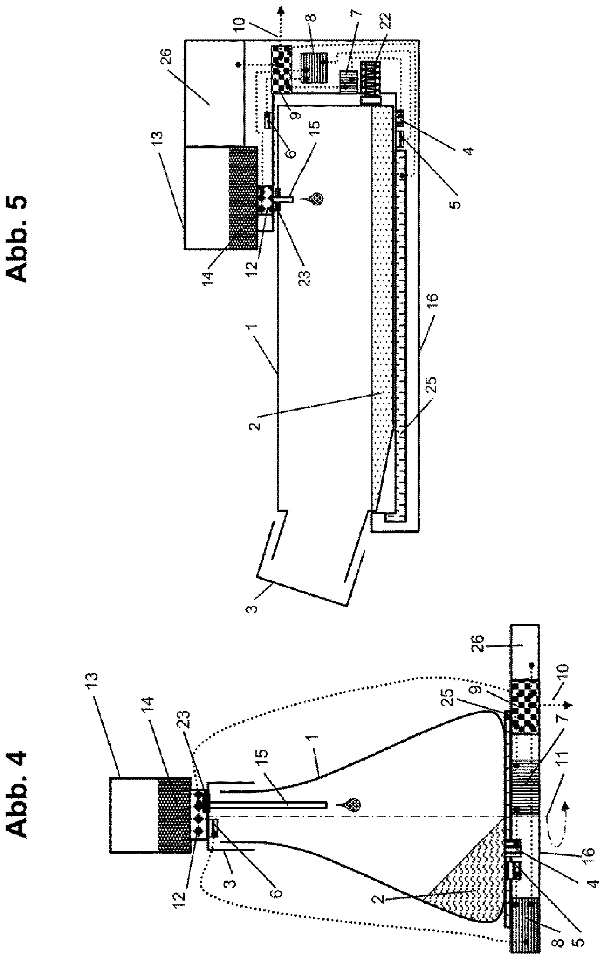

Abbildung 4 zeigt eine Ausführungsform der Vorrichtung zur Umsetzung des Verfahrens der Erfindung für orbitalgeschüttelte Schüttelkolben. Als Reaktor **1** kommt hier ein typischer Erlenmeyerkolben zum Einsatz, welcher das Reaktionsgemisch **2** enthält und welcher zusammen mit dem unter ihm platzierten Gehäuse **16** in eine handelsübliche Einspannvorrichtung oder einen Halter geklemmt werden kann. Im Gehäuse **16** befindet sich eine Lichtquelle **4** mitsamt Steuereinheit **7**, ein Lichtsensor **5** mit Auswertungs- und Steuereinheit **8**, sowie ein mit den Steuereinheiten **7/8** verbundener Rechner **9**. Rechner **9**, welcher in vorteilhafter Ausgestaltung der Erfindung von einem Mikroprozessor gebildet wird, ist zur Versorgung mit elektrischer Energie mit einem Akkumulator **26** verbunden. Dies gilt auch für alle anderen elektronischen Komponenten der Vorrichtung, allerdings wurde aus Gründen der Übersichtlichkeit auf die Einzeichnung der entsprechenden Verbindungen verzichtet. Weiterhin ist an Rechner **9** ein Temperierungssystem **25,** welches z.B. aus Temperatursensor, Peltier-Element und Regulationseinheit besteht, angeschlossen.

Ein zweiter Teil der Vorrichtung in Abbildung 4 ist am Deckel **3** des Reaktors **1** angebracht. Zur Detektion von transmittiertem und/oder vorwärts gestreutem Licht befindet sich im Deckel ein Lichtsensor **6** (aus Sterilitätsgründen gegebenenfalls hinter einer Glas- oder Kunststoffscheibe), welcher ebenso wie der an der Unterseite des Kolbens platzierte Lichtsensor **5** mit der Auswertungs- und Steuereinheit **8** verbunden ist. Auf dem Deckel befindet sich ein elektronisches Dosiersystem **12**, das mit dem Rechner **9** verbunden ist und von ihm gesteuert eine Komponente **14** aus Reservoir **13** über eine Zugabeleitung **15** in den Reaktor **1** und das Reaktionsgemisch **2** geben kann. Die Öffnung um die Zugabeleitung **15** wird durch ein Septum **23** steril abgedichtet.

Abbildung 5 bildet eine Ausführungsform der Vorrichtung zur Umsetzung des Verfahrens der Erfindung für wipp- oder orbitalgeschüttelte Kulturflaschen (T-Flasks) ab. Der Reaktor **1** wird hier durch eine mit Reaktionsgemisch **2** gefüllte T-Flask gebildet, welche mittels einer Einspannvorrichtung **22** am Gehäuse **16** fixiert wird. Analog zu Abbildung 4 befindet sich im Gehäuse **16** eine Lichtquelle **4** mitsamt Steuereinheit **7**, zwei gegenüberliegende Lichtsensoren **5/6** mit Auswertungs- und Steuereinheit **8**, ein mit den Steuereinheiten **7/8** verbundener Rechner **9**, sowie ein elektronisches Dosiersystem **12**, das ebenfalls mit Rechner **9** verbunden ist und von ihm gesteuert eine Komponente **14** aus Reservoir **13** über eine das Septum **23** durchdringende Zugabeleitung **15** in den Reaktor **1** und das Reaktionsgemisch **2** geben kann. Rechner **9**, welcher in vorteilhafter Ausgestaltung der Erfindung von einem Mikroprozessor gebildet wird, ist zur Versorgung mit elektrischer Energie mit einem Akkumulator **26** verbunden. Dies gilt auch für alle anderen elektronischen Komponenten der Vorrichtung, allerdings wurde aus Gründen der Übersichtlichkeit auf die Einzeichnung der entsprechenden Verbindungen verzichtet. Weiterhin ist an Rechner **9** ein unterhalb des Reaktors **1** angebrachtes Temperierungssystem **25**, welches z.B. aus Temperatursensor, Peltier-Element und Regulationseinheit besteht, angeschlossen.

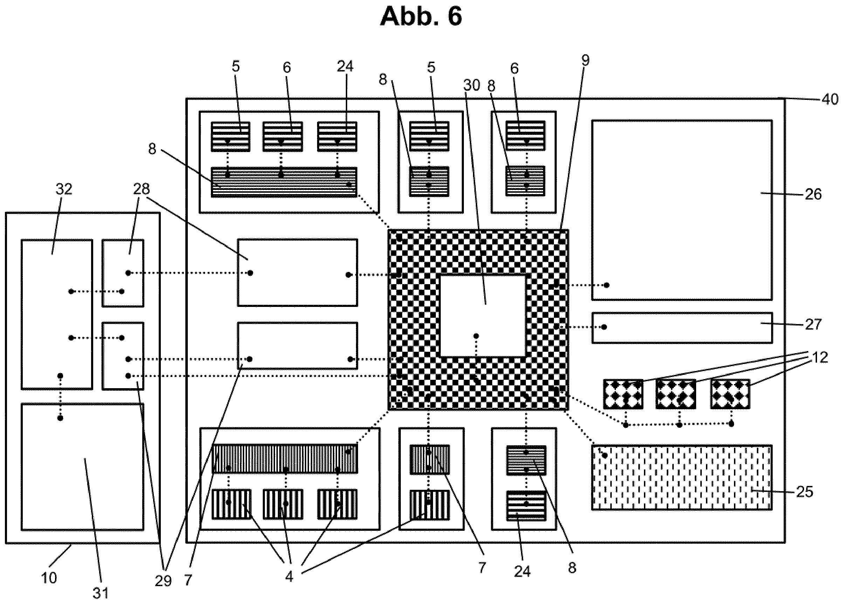

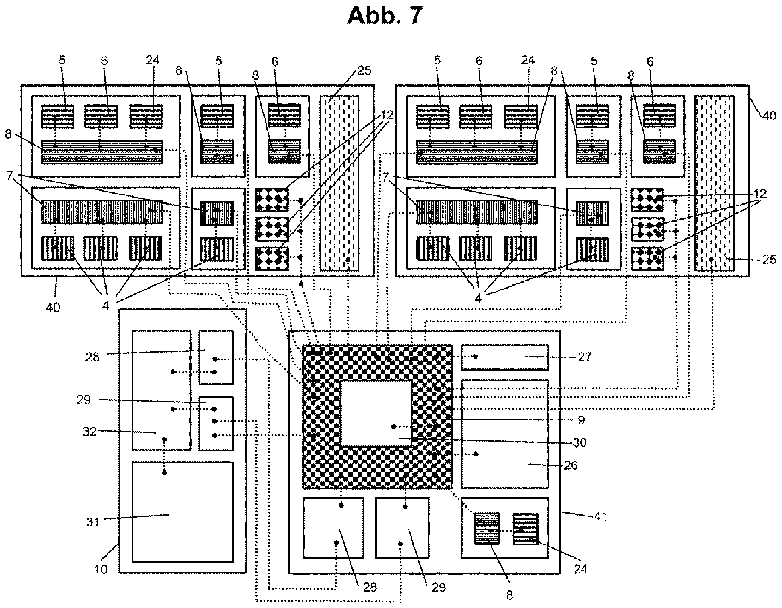

Die in Abbildung 4 und Abbildung 5 dargestellten Ausführungsformen der Erfindung sind nichtmodular und einzeln in Kombination mit einem Rechner **10** mit Benutzersoftware **31** funktionsfähig. Es kann jedoch vorteilhaft sein, die Vorrichtung zu modularisieren. Diesbezüglich zeigt Abbildung 6 die schematische Darstellung einer nichtmodularen Ausführung der Vorrichtung und Abbildung 7 die schematische Darstellung einer modularen Ausführung der Vorrichtung, um die Interaktion und Modularisierbarkeit der einzelnen Vorrichtungsbestandteile näher zu beschreiben. Die schematisch abgebildeten elektronischen Vorrichtungsbestandteile bilden dabei funktionelle Einheiten, welche nicht notwendigerweise als einzelne elektronische oder anderweitig technische Komponenten ausgeprägt sein müssen. Teilweise lassen sich mehrere funktionelle Einheiten in einer elektronischen Komponente (z.B. auf einem Chip) zusammenfassen.

Die in Abbildung 6 gezeigte nichtmodulare Ausführung setzt sich aus zwei Hauptbestandteilen zusammen, einer Messstation **40** und einem Rechner **10** mit Benutzersoftware **31**, wobei die Messstation neben den messtechnisch relevanten funktionellen Einheiten **(4/5/6/7/8/24)** und den das Reaktionsgemisch **2** beeinflussenden Einheiten **(12/25)** auch noch einen Rechner **9** mit Firmware **30** sowie je nach Ausführung verschiedene Kommunikationsmodule **28/29** und die elektrische Energieversorgung **26/27** umfasst. Zur Umsetzung der in den meisten Schüttelreaktoranwendungen erwünschten Parallelisierung kann es jedoch vorteilhaft sein, jede unmittelbar mit einem Reaktor **1** in Kontakt stehenden Messstation **40** so klein wie möglich zu halten. Die modularisierte Ausführung in Abbildung 7 ermöglicht dies durch Auslagerung einiger funktioneller Einheiten aus den Messstationen **40** in eine durch mehrere Messstationen **40** gemeinsam nutzbare Basisstation **41** und durch Beschränkung jeder Messstation **40** auf die unabkömmlich am Reaktor **1** benötigten funktionellen Einheiten.

Wie in Abbildung 7 dargestellt, können insbesondere Rechner **9** mit Firmware **30** sowie die elektrische Energieversorgung über einen Akkumulator **26** oder einen Netzanschluss **27** und die gegebenenfalls für die Kommunikation mit Rechner **10** und Benutzersoftware **31** benötigten Kommunikationsmodule **28/29** in eine von mehreren Messstationen **40** gemeinsam genutzte Basisstation **41** integriert werden. Jede Messstation **40** ist zur Stromversorgung und zur Kommunikation mit der Basisstation **41** verbunden, wobei die Kommunikation zwischen Rechner **9** mit Firmware **30** und den funktionellen Einheiten der Messstation (auch im nichtmodularen Aufbau) in vorteilhafter Ausgestaltung der Erfindung über serielle Kommunikationsprotokolle und - standards (z.B. SPI, $I^2C$, USB, CAN, Ethernet, IEEE 802-Standards, etc.) abläuft.

Die folgenden Ausführungen gelten sowohl für nichtmodulare (z.B. Abbildung 6) als auch für modulare (z.B. Abbildung 7) Ausführungsformen der Erfindung.

Die mit Rechner **9** und Firmware **30** kommunizierenden funktionellen Einheiten sind insbesondere aber nicht ausschließlich die Steuereinheit **7** für mindestens eine Lichtquelle **4** und die Steuer- und Auswertungseinheit **8** für mindestens einen Lichtsensor **5/6**, wobei eine Messstation **40** auch mehrere Steuer-/Auswertungseinheiten **7/8** sowie mehrere Lichtquellen **4** und Lichtsensoren **5/6** enthalten kann. Hauptkomponenten der Steuereinheit **7** für mindestens eine Lichtquelle **4** können insbesondere aber nicht ausschließlich Digitalpotentiometer, Diodentreiber, Lasertreiber und Mikroprozessoren sein, welche genutzt werden können, um Eigenschaften wie Intensität oder Spektralbereiche des von Lichtquelle **4** abgestrahlten Lichtes **17** zu modifizieren und einzustellen.

Aufgabe der Steuer- und Auswertungseinheit **8** für mindestens einen Lichtsensor **5/6** ist die Digitalisierung der vom Lichtsensor **5/6** erzeugten analogen Messsignale, die Verstärkung und Filterung der vom Lichtsensor **5/6** erzeugten analogen Messsignale vor der Digitalisierung und die Übergabe der digitalisierten Messdaten an den Rechner **9** und die Firmware **30**. Hauptkomponenten der Steuer- und Auswertungseinheit **8** für mindestens einen Lichtsensor **5/6** können insbesondere aber nicht ausschließlich Digitalpotentiometer, Operationsverstärker, Analog-Digital-Converter, geeignete Frequenzfilter und Mikroprozessoren sein.

In einigen Ausführungsformen können Lichtquellen **4** und Lichtsensoren **5/6** einzeln oder gemeinsam oder gemeinsam mit der zugehörigen Steuer-/Auswertungseinheit **7/8** verfahrbar sein, was insbesondere aber nicht ausschließlich von Rechner **9** mit Firmware **30** angesteuerte Elektromotoren oder andere elektrische Magnetsysteme ausführen können. Zweck der Verfahrbarkeit ist die Anpassung der Positionen von Lichtquellen **4** und Lichtsensoren **5/6,** um die Vorrichtung automatisiert an die jeweiligen Prozess- und Messerfordernisse anzupassen.

Weiterhin können, wenn erforderlich, mindestens ein Temperierungssystem **25** und mindestens ein Dosiersystem **12** in die Messstation integriert werden, welche beide über Rechner **9** mit Firmware **30** angesteuert werden, um rückgekoppelt auf die Messdaten und die durch Rechner **10** und Benutzersoftware **31** ermittelten weiteren Parameter und Eigenschaften des Prozesses in den Prozess eingreifen zu können. Hauptkomponenten des Temperierungssystems **25** können insbesondere aber nicht ausschließlich Temperatursensoren, Peltier-Elemente, Analog-Digital-Converter und Mikroprozessoren sein. Hauptkomponenten des Dosiersystem **12** können insbesondere aber nicht ausschließlich elektrisch steuerbare Ventile und Pumpen, Temperatursensoren, Flüssigkeitssensoren, Drucksensoren, Peltier-Elemente, Analog-Digital-Converter und Mikroprozessoren sein.

Sowohl in mindestens einer Messstation **40** als auch in mindestens einer Basisstation **41** kann mindestens ein Umgebungslichtsensor **24** mit entsprechender Steuer- und Auswertungseinheit **8** integriert sein, um Beeinflussungen der optischen Messungen an Reaktor(en) **1** und Reaktionsgemisch(en) **2** zu erkennen und entsprechende Interferenzen in den Messdaten eliminieren oder reduzieren zu können.

In vorteilhafter Ausgestaltung der Erfindung erfolgt auch die Kommunikation zwischen Rechner **10** und Benutzersoftware **31** und Rechner **9** mit Firmware **30** über serielle Kommunikationsprotokolle und -standards (z.B. SPI, I$^2$C, USB, CAN, Ethernet, IEEE 802-Standards, etc.). Gegebenenfalls dazu benötigte Kommunikationsmodule **28/29** befinden sich je nach Ausführung auf Rechner **10** und an Rechner **9** der Messstation **40** oder Basisstation **41.**

In vorteilhafter Ausgestaltung der Erfindung ist die Hardware **32** von Rechner **10** deutlich leistungsfähiger als Rechner **9,** sodass in der Benutzersoftware **31** deutlich komplexere und ressourcenintensivere mathematische Verfahren und informationstechnische Algorithmen implementiert werden können als in Firmware **30,** welche primär der Ansteuerung der elektronischen Komponenten auf Mess- und/oder Basisstation **40/41** dient.

Abbildung 8 veranschaulicht den Einsatz von Sensorarrays als Lichtsensor **5/6** am Beispiel eines orbitalgeschüttelten Kolbens als Reaktor **1.** Das von einer Lichtquelle **4** in Reaktor 1 und Reaktionsgemisch **2** eingestrahlte Licht **17** wird an mindestens einer Komponente des Reaktionsgemisches **2** gestreut. Die Streuung erfolgt kontinuierlich über einen bestimmten Streuwinkelbereich, meist jedoch in alle Raumrichtungen. Die Intensität des gestreuten Lichtes **18** ist abhängig vom Streuwinkel. Diese Abhängigkeit kann genutzt werden, um in einem breiten, mehrere Größenordnungen umfassenden Bereich der optischen Dichte des Reaktionsgemisches **2** sehr genaue und verlässliche Messungen durchzuführen. Dazu werden viele einzelne Lichtsensoren **5** zu einem Lichtsensor-Array **33** zusammengefasst, welches simultan die Streulichtintensität bei verschiedenen Streuwinkeln erfasst. Der Vergleich und die Korrelation der bei verschiedenen Zeiten aufgenommenen winkelabhängigen Streulichtintensitäten gewährleisten verlässliche Messungen über mehrere Größenordnungen der optischen Dichte. Diese Verlässlichkeit kann insbesondere aber nicht ausschließlich erreicht werden durch die gezielte Auswahl des jeweils optimalsten Lichtsensors **5** im Lichtsensor-Array **33,** sowie durch den Vergleich und die Gewichtung der Werte aller Lichtsensoren **5** im Lichtsensor-Array **33** mittels geeigneter mathematischer Verfahren. Derartige Lichtsensor-Arrays **33** können auch als Transmissionssensoren eingesetzt werden, insbesondere aber nicht ausschließlich um die Transmission in verschiedenen Raumrichtungen simultan zu erfassen.

Als Lichtsensor-Array **33** können insbesondere aber nicht ausschließlich integrierte eindimensionale und zweidimensionale Lichtsensor-Arrays wie CCD-Chips oder CMOS-APS-Chips, sowie andere eindimensionale und zweidimensionale Arrays von Fotodioden, Fotowiderständen und Fototransistoren eingesetzt werden.

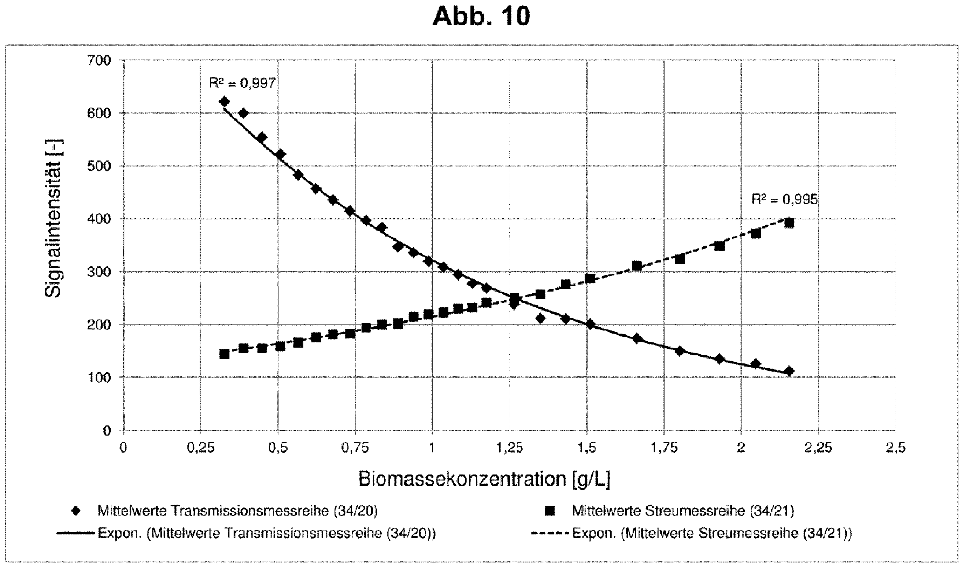

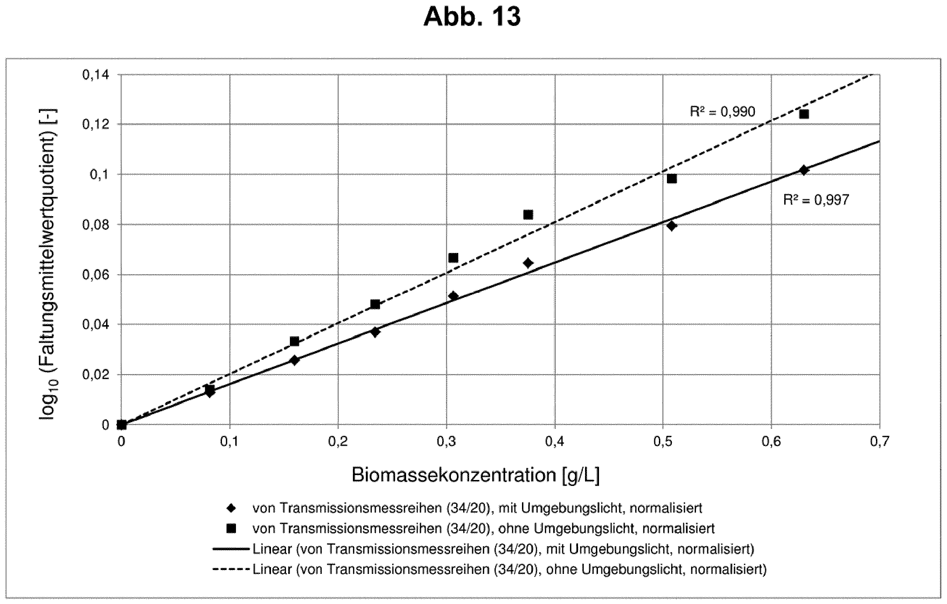

Die Abbildungen 10 bis 14 zeigen beispielhafte Messergebnisse, die mittels des erfindungsgemäßen Verfahrens ermittelt wurden. Die Messungen erfolgten mit einer Vorrichtung nach Abbildung 2 in mit 160 Umdrehungen pro Minute orbitalgeschüttelten Erlenmeyerkolben als Reaktor **1.** Als Reaktionsgemisch **2** kamen Suspensionen von Bäckerhefezellen (*S. cerevisiae*) in LB-Medium zum Einsatz, wobei für die Messungen zu Abbildung 10 bis 12 relative Kolbenfüllvolumina von 20% und für die Messungen zu Abbildung 13 bis 14 relative Kolbenfüllvolumina von 10% verwendet wurden. Als Lichtquelle **4** wurde eine LED mit einer Peak-Wellenlänge von 528 nm genutzt, welche unterhalb des Kolbens nichtzentral platziert war (ähnlich Abbildung 2). Als Lichtsensoren **5/6** kamen Fotodioden zum Einsatz, wobei jeweils eine Fotodiode als Lichtsensor **5** für Streulicht unter dem Kolben neben der Lichtquelle **4** und eine Fotodiode als Lichtsensor **6** für transmittiertes Licht im Deckel **3** des Kolbens platziert war. Die Lichtintensität $I_0$ vor Durchquerung des Reaktors und des Reaktionsgemisches wurde nicht gemessen. Die im Folgenden, unter anderem anhand der Abbildungen 10 bis 14, eingeführten Formeln und Gleichungen für die Zusammenhänge zwischen Biomassekonzentration und Signalintensitäten oder anderen Ergebnissen optischer Messungen gelten in gleicher oder abgewandelter Form für viele weitere mit eingestrahltem Licht **17** interagierende Komponenten möglicher Reaktionsgemische **2,** insbesondere aber nicht ausschließlich für Konzentrationen von Organismen, Proteinen, Nukleinsäuren (z.B. DNA, RNA), Lipiden, Zuckern, Biopolymeren, Kunststoffen, sowie anderen organischen und/oder anorganischen Partikeln, Molekülen, Ionen und, allgemein, Substanzen.

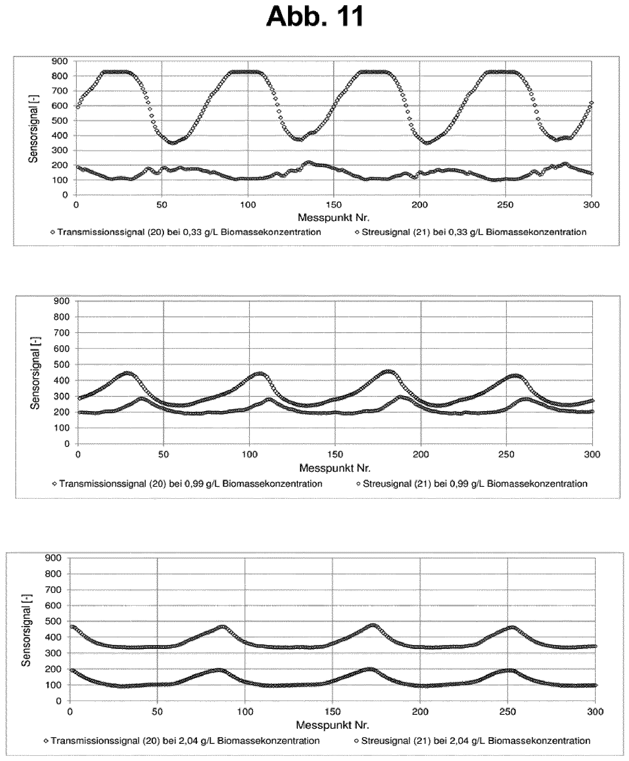

Abbildung 10 verdeutlicht die Korrelation zwischen der Intensität des Signals der Lichtsensoren **5/6** und der Biomassekonzentration im Reaktionsgemisch **2**. Dabei stellt ein Signalintensitätswert den Mittelwert jeweils einer Transmissions- bzw. Streumessreihe **34/20** bzw. **34/21** dar, wie sie exemplarisch in Abbildung 11 für die Signalintensitäten aus Abbildung 10 bei den Biomassekonzentrationen 0,33 g/L, 0,99 g/L und 2,04 g/L gezeigt sind. Infolge der erfindungsgemäßen Erfassung des Lichtes durch die Lichtsensoren mit einer Frequenz, sodass die Schüttelfrequenz kein ganzzahliges Vielfaches der Erfassungsfrequenz ist, ergibt sich erfindungsgemäß ein schüttelbedingt periodisch schwankendes Sensorsignal als Folge des periodisch schwankenden Füllstandes. Für die in Abbildung 10-13 dargestellten Beispielmessung wurde eine Messfrequenz von 200 Hz gewählt, sodass eine Schüttelperiode durch 75 Messpunkte abgebildet wird.

Aus Abbildung 10 ist sowohl für die Transmissionswerte als auch für die Streuwerte ein klarer Zusammenhang zwischen der als Mittelwert einer Messreihe ermittelten Signalintensität und der Biomassekonzentration des Reaktionsgemisches zu erkennen. Die abgebildeten Signalintensitäten von Transmissions- und Streulicht stehen im gezeigten Messbereich jeweils in einem exponentiellen Zusammenhang mit der Biomassekonzentration, wie es auch für die Absorption von Licht in homogenen verdünnten Lösungen allgemein bekannt ist. Das entsprechende Gesetz für die Transmission von Licht nach Lambert und Beer lautet

$$E = log_{10}\left(\frac{I_0}{I}\right) = \varepsilon \cdot c \cdot d$$

mit E der optischen Dichte, $I_0$ der Lichtintensität vor Durchquerung des Mediums, $I$ der Lichtintensität nach Durchquerung des Mediums, $\varepsilon$ dem Extinktionskoeffizient, c der Konzentration der absorbierenden Komponente und d dem durchstrahlten Lichtweg, welcher hier aufgrund der erfindungsgemäßen periodischen Lichtwegschwankungen durch den (gegebenenfalls gewichteten) Mittelwert aller Lichtwege gebildet wird.

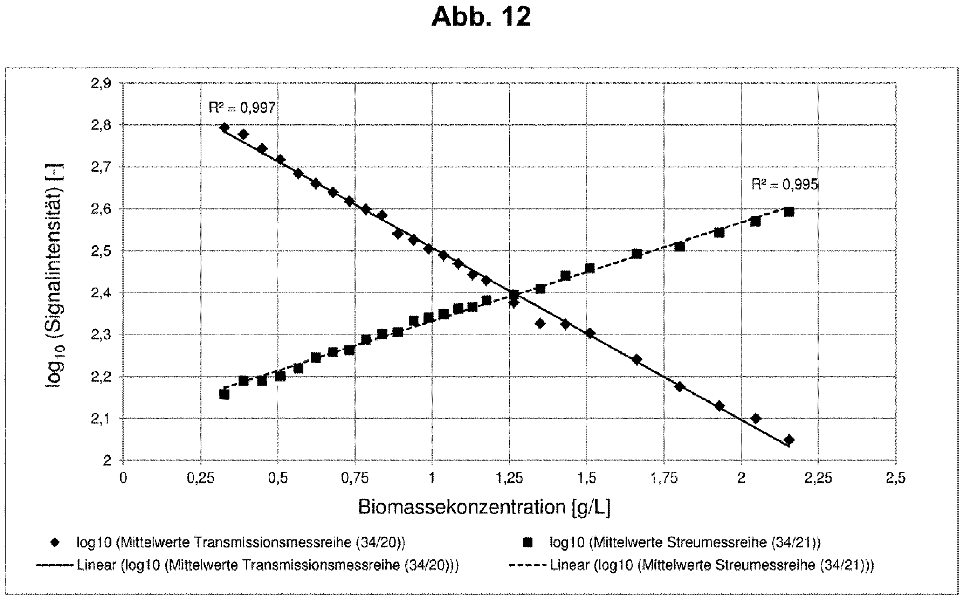

Der lineare Zusammenhang zwischen optischer Dichte und der absorbierenden Komponente des Reaktionsgemisches gilt auch für den Logarithmus der Signalintensitäten und die Biomasse, wie aus Abbildung 12 hervorgeht, da angenommen werden kann, dass $I_0$ für alle Messungen innerhalb eines Experimentes konstant ist. Ist $I_0$ nicht bekannt, so kann aus den Änderungen der Signalintensität nicht nur die Änderung der optischen Dichte und der Biomassekonzentration ermittelt werden, sondern auch die absolute Biomassekonzentration, sofern die Biomassekonzentration für einen Signalintensitätswert bekannt ist. Dies ergibt sich für Transmissionssignale aus der Differenz der optischen Dichten und der Umstellung des Lambert-Beer-Gesetzes.

$$\Delta E = E_2 - E_1 = log_{10}\left(\frac{I_1}{I_2}\right) = \varepsilon \cdot d \cdot (c_2 - c_1)$$

$$c_2 = \frac{log_{10}\left(\frac{I_1}{I_2}\right)}{\varepsilon \cdot d} + c_1$$

Zur derartigen Bestimmung absoluter Biomassekonzentrationen müssen $\varepsilon$ der Extinktionskoeffizient und $d$ der durchstrahlte Lichtweg bekannt sein, welche aus Eichreihen ermittelt werden können. Sind $\varepsilon$ und $d$ nicht bekannt, so können absolute Biomassekonzentrationen auch bestimmt werden, sofern die Biomassekonzentration für einen Signalintensitätswert bekannt ist und sofern $I_0$ bekannt ist (insbesondere aber nicht ausschließlich indem $I_0$ während des Experimentes gemessen wird, oder indem notwendige $I_0$-Werte durch den Produkthersteller ermittelt und im Gerät eingespeichert wurden). Dies ergibt sich aus der Änderung der relativen Biomassekonzentration $c_2/c_1$.

$$\frac{c_2}{c_1} = \frac{\frac{log_{10}\left(\frac{I_1}{I_2}\right)}{\varepsilon \cdot d} + c_1}{c_1} = \frac{log_{10}\left(\frac{I_1}{I_2}\right)}{\varepsilon \cdot d} \cdot \frac{1}{c_1} + 1 = \frac{log_{10}\left(\frac{I_1}{I_2}\right)}{\varepsilon \cdot d} \cdot \frac{\varepsilon \cdot d}{log_{10}\left(\frac{I_0}{I_1}\right)} + 1 = \frac{log_{10}\left(\frac{I_1}{I_2}\right)}{log_{10}\left(\frac{I_0}{I_1}\right)} + 1$$

$$c_2 = \left( \frac{log_{10}\left(\frac{I_1}{I_2}\right)}{log_{10}\left(\frac{I_0}{I_1}\right)} + 1 \right) \cdot c_1$$

Für die Ermittlung der Biomassekonzentration aus Streusignalen kann das Lambert-Beer-Gesetz nicht eingesetzt werden. Allerdings lässt sich, wie aus Abbildung 10/12 hervorgeht, auch die Abhängigkeit zwischen Streusignal und Biomassekonzentration im gezeigten Messbereich exponentiell modellieren, sodass analog zu den obigen Gleichungen für die Streuintensität $I_s$ formuliert werden kann

$$I_s = I_0 \cdot 10^{\sigma \cdot c \cdot V}$$

mit $I_0$ der Lichtintensität vor Durchquerung des Mediums, $\sigma$ dem Streukoeffizient, c der Konzentration der streuenden Komponente und $V$ dem detektierbares Streulicht erzeugenden Reaktionsvolumen, welches hier aufgrund der erfindungsgemäßen periodischen Lichtwegschwankungen durch den (gegebenenfalls gewichteten) Mittelwert aller $V$ gebildet wird. Infolge von Mehrfachstreuung entstammt das durch einen Streulichtsensor **5** detektierte Licht nicht notwendigerweise aus dem insgesamt vom Licht durchstrahlten Volumen. Vielmehr ist das detektierbares Streulicht erzeugende Reaktionsvolumen $V$ insbesondere bei höheren Biomassekonzentrationen abhängig von der jeweiligen Biomassekonzentration, sodass sich insgesamt bei entsprechenden Messbedingungen ein linear abschätzbarer Zusammenhang zwischen Streuintensität / und Biomassekonzentration ergibt, der formuliert werden kann als

$$I_s = I_0 \cdot \sigma_{lin} \cdot c$$

mit $I_0$ der Lichtintensität vor Durchquerung des Mediums, $\sigma_{lin}$ dem linearen Streukoeffizient und c der Konzentration der streuenden Komponente. Bei niedrigen Biomassekonzentrationen ergibt sich daher analog zur Transmissionsmessung die Biomassekonzentration zu

$$c_2 = \frac{log_{10}\left(\frac{I_{s2}}{I_{s1}}\right)}{\sigma \cdot V} + c_1$$

oder zu

$$c_2 = \left( \frac{log_{10}\left(\frac{I_{s2}}{I_{s1}}\right)}{log_{10}\left(\frac{I_{s1}}{I_0}\right)} + 1 \right) \cdot c_1$$

Für höhere Biomassekonzentrationen kann die Biomassekonzentration abgeschätzt werden zu

$$c = \frac{I_s}{I_0 \cdot \sigma_{lin}}$$

oder zu

$$c_2 = \frac{I_{s2}}{I_{s1}} \cdot c_1$$

Zur korrekten Auswertung der erfindungsgemäß periodisch schwankenden Messreihen **34** bieten sich neben der oben angewendeten Methode der Mittelwertbildung weitere mathematische Verfahren an, insbesondere aber nicht ausschließlich das direkte Fitting periodischer Modellfunktionen auf die Messreihen **34** oder statistische Auswertungen jeder Messreihe **34** über Klassifizierungen, das Fitting von Verteilungsfunktionen, Korrelationsanalysen zwischen verschiedenen Messreihen eines Lichtsensors **5/6** und/oder verschiedener Lichtsensoren **5/6** etc. Ein weiteres mathematisches Verfahren, welches insbesondere zur robusten Auswertung der Messreihen bei geringen Biomassekonzentrationen geeignet ist, ist die diskrete Faltung zweier zu unterschiedlichen Zeitpunkten ermittelten Messreihen, welche jeweils in einer diskreten Faltungsfunktion $F$ für das gefaltete Messreihenpaar resultiert. Diese Faltungsfunktion kann nun weiter mathematisch ausgewertet und mit der Biomasse korreliert werden. Eine mögliche Art der Auswertung ist die Bildung des Mittelwertes aller Elemente einer diskreten Faltungsfunktion. Abbildung 13 zeigt für Transmissionsmessungen die Korrelation zwischen dem dekadischen Logarithmus des Quotienten der Faltungsmittelwerte und der Biomassekonzentration. Analog zu den obigen Ausführungen zur Auswertung der Transmissionsmessreihen ergibt sich dieser Zusammenhang nach der Gleichung

$$\Delta E_F = log_{10}\left(\frac{\overline{F}_1}{\overline{F}_2}\right) = \varepsilon_F \cdot d \cdot (c_2 - c_1)$$

mit $\Delta E_F$ der Änderung der faltungsspezifischen optischen Dichte, $\overline{F}_1$ und $\overline{F}_2$ den Mittelwerten der Faltungsfunktionen $F_1$ und $F_2$, $\varepsilon_F$ dem faltungsspezifischen Extinktionskoeffizient, $c$ der Konzentration der absorbierenden Komponente und $d$ dem durchstrahlten Lichtweg. Daraus ergibt sich bei mindestens einem bekannten Konzentrationswert die Biomassekonzentration zu

$$c_2 = \frac{log_{10}\left(\frac{\overline{F}_1}{\overline{F}_2}\right)}{\varepsilon_F \cdot d} + c_1$$

Abbildung 13 zeigt weiterhin, dass die Erfindung sowohl unter Ausschluss als auch in Anwesenheit von Umgebungslicht (insbesondere Tageslicht und Raumbeleuchtung) bis in geringe Biomassekonzentrationsbereiche robuste Messungen ermöglicht. Die Messdaten mit Umgebungslicht wurden bei Tageslicht und zusätzlicher Zimmerbeleuchtung durchgeführt, während die Messungen ohne Umgebungslicht durch Abdunkelung des gesamten Messaufbaus mittels eines schwarzen, lichtundurchlässigen Gewebes realisiert wurden. Beide Messungen zeigen auch bei niedrigen Biomassekonzentrationen einen linearen Zusammenhang zwischen Biomassekonzentration und dem dekadischen Logarithmus der Faltungsmittelwertquotienten (linearer Zusammenhang gilt auch für die nichtdargestellten Signalmittelwerte). Einzig die Steigung der linearen Funktionen ist unterschiedlich, mit Umgebungslicht steigt das verarbeitete Signal langsamer an, da die Höhe der erfindungsgemäßen periodischen Schwankungen des Transmissionssignals **20** infolge der höheren Hintergrundlichtintensität geringer ausfällt als für die abgedunkelte Messung. Allerdings kann die entsprechende Skalierung der Steigung auf die Umgebungslichtverhältnisse durch Verwendung von mindestens einem parallel zur Messung an Reaktor **1** und Reaktionsgemisch **2** ausgelesenen Umgebungslichtsensor **24** erreicht werden, sodass auch bei wechselndem Umgebungslicht robuste Messungen möglich sind.

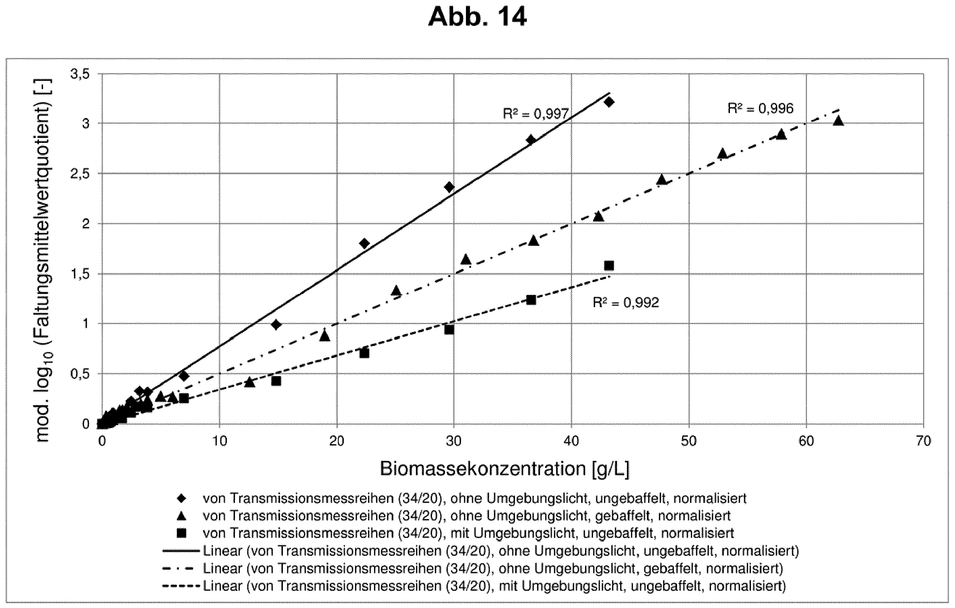

Abbildung 14 zeigt den Zusammenhang zwischen Biomassekonzentration und einem modifizierten dekadischen Logarithmus der Faltungsmittelwertquotienten von Transmissionsmessreihen über einen weiten Konzentrationsbereich bis über 40 g/L. Dargestellt sind Ergebnisse für zwei Messungen an ungebaffelten Kolben, jeweils eine Messung mit und eine ohne Umgebungslicht, sowie eine Messung an einem gebaffelten Kolben ohne Umgebungslicht. Die Messfrequenz an den ungebaffelten Kolben betrug 200 Hz, wohingegen Messreihen für den gebaffelten Kolben mit 450 Hz aufgezeichnet wurden, um die turbulente Verteilung des Reaktionsgemisches **2** im Reaktor **1** besser erfassen zu können.

Über den in Abbildung 14 dargestellten Biomassekonzentrationsbereich ist der Zusammenhang zwischen Transmissionsintensität und Biomassekonzentration nicht mehr mit einer einfachen Exponentialfunktion modellierbar, sodass das Lambert-Beer-Gesetz keine Gültigkeit mehr hat, sondern modifiziert werden muss. Optimal beschreiben lässt sich dieser Zusammenhang über die Summe von mindestens zwei Exponentialfunktionen mit unterschiedlichen Koeffizienten im Exponent, beispielsweise

$$I = I_0 \cdot \left(a \cdot 10^{-\varepsilon_1 \cdot c \cdot d} + b \cdot 10^{-\varepsilon_2 \cdot c \cdot d}\right)$$

Da derartige Funktionen üblicherweise analytisch nicht nach der Konzentration c aufgelöst werden können, muss die Biomassekonzentration durch Verwendung einer analytisch lösbaren Abschätzungsfunktion, welche den Verlauf der Summe mehrerer Exponentialfunktionen hinreichend genau abbildet, ermittelt werden. Zu diesem Zweck kann beispielsweise die dem Lambert-Beer-Gesetz zugrundeliegende Funktion modifiziert werden zu

$$I = I_0 \cdot 10^{-\varepsilon \cdot c^Z \cdot d}$$

woraus sich die Biomassekonzentration c ergibt als

$$c = \sqrt[z]{\frac{log_{10}\left(\frac{I_0}{I}\right)}{\varepsilon_z \cdot d}}$$

mit $I_0$ der Lichtintensität vor Durchquerung des Mediums, $I$ der Lichtintensität nach Durchquerung des Mediums, $\varepsilon_z$ dem korrigierten Extinktionskoeffizient, z dem Korrekturexponent und d dem durchstrahlten Lichtweg. Analog zu den obigen Gleichungen zur Transmission lässt sich die Biomassekonzentration dann auch berechnen zu

$$c_2 = \sqrt[z]{\frac{log_{10}\left(\frac{I_1}{I_2}\right)}{\varepsilon_z \cdot d} + c_1^z}$$

Wie für die vorangehende Beispielmessung gezeigt, können für die Intensitäten auch die Faltungsmittelwerte und für die korrigierten Extinktionskoeffizienten auch die korrigierten faltungsspezifischen Extinktionskoeffizienten eingesetzt werden. Die Anwendung eines derartig modifizierten Lambert-Beer-Gesetzes erlaubt die Bestimmung der Biomassekonzentration über einen weiten Messbereich ausschließlich unter Nutzung von Transmissionsdaten. Durch geeignete Kombination der Messdaten mehrerer Lichtsensoren **5/6** kann die Robustheit und Genauigkeit der Messungen zusätzlich verbessert werden.

Abbildung 14 demonstriert zudem die Einsetzbarkeit der Erfindung an kontinuierlich geschüttelten gebaffelten Systemen, die durch turbulente Strömungen, inhomogene Reaktionsgemischverteilungen im Reaktor und Störfaktoren wie Blasen und Schaum charakterisiert sind. Die nach dem Stand der Technik bekannten Technologien sind nicht in der Lage, an solchen Systemen robuste Messungen im laufenden Schüttelbetrieb durchzuführen. Dies wird erst durch die erfindungsgemäße Aufnahme einer Vielzahl von Messwerten mit einer Frequenz, sodass die Schüttelfrequenz kein ganzzahliges Vielfaches der Erfassungsfrequenz ist, ermöglicht.

**Liste der Bezugszeichen**

1    Reaktor
2    Reaktionsgemisch
3    Deckel
4    Lichtquelle
5    Lichtsensor für Streulicht
6    Lichtsensor für transmittiertes und/oder vorwärtsgestreutes Licht
7    Steuereinheit für mindestens eine Lichtquelle **4**
8    Auswertungs- und Steuereinheit für mindestens einen Lichtsensor **5/6**
9    Rechner (z.B. mit zentraler Regel- und Steuereinheit als Mikrocontroller oder SoC)
10    Rechner (z.B. als PC oder Server)
11    Schüttelachse orthogonal zu Schüttelebene bei Orbitalschütteln
12    Dosiersystem
13    Reservoir
14    Zuzugebende Komponente
15    Zugabeleitung
16    Gehäuse
17    Strahl von Lichtquelle ausgesandtem Licht
18    Strahl von rückgestreutem Licht
19    Strahl von transmittiertem und/oder vorwärts gestreutem Licht
20    Signal von 6 ("Transmissionssignal")

21      Signal von 5 ("Streusignal")
22      Einspannvorrichtung
23      Septum
24      Umgebungslichtsensor
25      Temperierungssystem
26      Akkumulator
27      Netzgebundene Stromversorgung
28      Funkmodul
29      Kabelgebundenes Kommunikationsmodul
30      Firmware
31      Benutzersoftware mit graphischer Oberfläche
32      Rechner-Hardware
33      Lichtsensor-Array
34      Messreihe(n)
35      Zeitreihe(n)
36      Mathematische und/oder informationstechnische Verfahren/Algorithmen
37      Parameter und Eigenschaften des Prozesses
38      Optik zur Modifikation des Lichtes von Lichtquelle **4**
39      Optik zur Modifikation des Lichtes aus Reaktionsgemisch **2** und Reaktor **1**
40      Messstation
41      Basisstation

**Patentansprüche**

1.  Verfahren zur Bestimmung der optischen Dichte und/oder der Veränderung der optischen Dichte eines Reaktionsgemisches (2) in einem geschüttelten Reaktor (1),
    wobei

        - Licht von mindestens einer Lichtquelle (4) in das Reaktionsgemisch (2) gelangt und
        - das das Reaktionsgemisch (2) verlassende Licht durch mindestens einen Lichtsensor (5,6) erfasst wird, sodass sowohl eine Transmission durch als auch eine Streuung an sich im Lichtpfad befindlicher Materie durch verschiedene Lichtquelle-Lichtsensor-Paare gemessen werden, und

        - während der Erfassung des Lichtes durch den mindestens einen Lichtsensor (5,6) der Reaktor (1) und das Reaktionsgemisch (2) geschüttelt werden und
        - die Erfassung des Lichtes durch den mindestens einen Lichtsensor (5,6) mit einer Frequenz erfolgt, sodass die Schüttelfrequenz kein ganzzahliges Vielfaches der Erfassungsfrequenz ist,
        - wobei zwischen dem Reaktor und mindestens einem Lichtsensor (5,6) keine Relativbewegung stattfindet und
        - für die Transmission und für die Streuung jeweils mehrere in einem bestimmten zeitlichen Intervall von mindestens einem Lichtsensor (5,6) erfassten Messpunkte (20,21) zu einer Messreihe (34) zusammengefasst werden,

    wobei jede Messreihe (34) eine schüttelbedingt schwankende Kurve ist, deren Form durch periodisch schwankende Höhen des Reaktionsgemisches im jeweiligen Lichtpfad verursacht wird,
    und wobei die Messreihen (34) der Transmission und der Streuung zur Bestimmung der optischen Dichten und/oder der Veränderung der optischen Dichte genutzt werden.

2.  Verfahren nach Anspruch 1, **dadurch gekennzeichnet, dass**

        - die Erfassung des Lichtes durch den mindestens einen Lichtsensor (5,6) mit einer Frequenz erfolgt, die höher ist, als die Schüttelfrequenz und/oder
        - mindestens ein Lichtpfad mindestens eines Lichtquelle-Lichtsensor-Paares (4-5,6) nicht parallel zu einer Schüttelachse und/oder Schüttelebene ausgerichtet ist.

3.  Verfahren nach einem der Ansprüche 1 bis 2, **dadurch gekennzeichnet, dass** aus den in einem bestimmten zeitlichen Intervall erfassten Messreihen (34) der Transmission und der Streuung mittels mindestens eines geeig-

18

neten mathematischen/informationstechnischen Verfahrens (36) und/oder mindestens einer Modellfunktion die optische Dichte und/oder die Veränderung der optischen Dichte und/oder mindestens ein fluidmechanischer Parameter und/oder mindestens eine fluidmechanische Eigenschaft des Reaktionsgemisches (2) im gemessenen Zeitintervall bestimmt wird.

**4.** Verfahren nach einem der Ansprüche 1 bis 3, **dadurch gekennzeichnet, dass** aus mindestens einer Messreihe (34) und/oder mindestens einer Zeitreihe (35) eines aus mindestens einem Messpunkt (20,21) und/oder mindestens einer Messreihe (34) bestimmten Parameters des Reaktionsgemisches (2) mindestens ein weiterer Parameter des Reaktionsgemisches (2) mittels mindestens eines geeigneten mathematischen/informationstechnischen Verfahrens (36) und/oder mindestens einer Modellfunktion bestimmt wird.

**5.** Verfahren nach einem der Ansprüche 1 bis 4, **dadurch gekennzeichnet, dass** mindestens eine Modellfunktion unter Verwendung mindestens eines geeigneten mathematischen Verfahrens (36) und/oder mindestens eines geeigneten informationstechnischen Algorithmus (36) neu generiert und/oder modifiziert wird.

**6.** Verfahren nach einem der Ansprüche 1 bis 5, **dadurch gekennzeichnet, dass**

- mindestens ein Dosiersystem (12) zur Zugabe mindestens eines Stoffes in Abhängigkeit der optischen Dichte und/oder der Veränderung der optischen Dichte und/oder mindestens eines anderen Prozessparameters gesteuert wird, und/oder
- mindestens ein weiteres den Prozess beeinflussendes technisches System, insbesondere ein System zur Temperierung (25) des Reaktionsgemisches (2), in Abhängigkeit der optischen Dichte und/oder der Veränderung der optischen Dichte und/oder mindestens eines anderen Prozessparameters gesteuert wird.

**7.** Vorrichtung zur Durchführung des Verfahrens nach einem der Ansprüche 1 bis 6, wobei

- die Vorrichtung mindestens eine Lichtquelle (4) umfasst, welche so positioniert und ausgerichtet ist, dass das von ihr abgestrahlte Licht unter den Bedingungen mindestens einer Schüttelart in den Reaktor (1) und direkt und/oder indirekt in das Reaktionsgemisch (2) gelangen kann und
- die Vorrichtung mindestens einen Lichtsensor (5,6) umfasst, wobei mindestens ein Lichtsensor (5,6) so positioniert und ausgerichtet ist, dass er direkt und/oder indirekt aus dem Reaktor (1) und/oder dem Reaktionsgemisch (2) ausgestrahltes Licht erfassen kann,
sodass sowohl eine Transmission durch als auch eine Streuung an sich im Lichtpfad befindlicher Materie durch verschiedene Lichtquelle-Lichtsensor-Paare gemessen werden können, und

- die Vorrichtung eine Schütteleinrichtung umfasst, und und
- die Vorrichtung mindestens einen Rechner (9,10) umfasst,

der Rechner (9,10) zu mindestens einem der folgenden Zwecke eingesetzt wird: Aufzeichnung der Messwerte, Speicherung der Messwerte, Verarbeitung der Messwerte, Darstellung der Messwerte und/oder Verarbeitungsergebnisse,
wobei die Vorrichtung so eingerichtet ist, dass:

- während der Erfassung des Lichtes durch den mindestens einen Lichtsensor (5,6) der Reaktor (1) und das Reaktionsgemisch (2) geschüttelt werden,
- die Erfassung des Lichtes durch den mindestens einen Lichtsensor (5,6) mit einer Frequenz erfolgt, sodass die Schüttelfrequenz kein ganzzahliges Vielfaches der Erfassungsfrequenz ist,
- zwischen dem Reaktor und dem mindestens einen Lichtsensor (5,6) keine Relativbewegung stattfindet,

für die Transmission und für die Streuung jeweils mehrere in einem bestimmten zeitlichen Intervall von dem mindestens einen Lichtsensor (5,6) erfassten Messpunkte (20,21) zu einer Messreihe (34) zusammengefasst werden, jede Messreihe eine schüttelbedingt schwankende Kurve ist, deren Form durch periodisch schwankende Höhen des Reaktionsgemisches im jeweiligen Lichtpfad verursacht wird, und

- die Messreihen (34) der Transmission und der Streuung zur Bestimmung der optischen Dichten und/oder der Veränderung der optischen Dichte genutzt werden.

**8.** Vorrichtung nach Anspruch 7, **dadurch gekennzeichnet, dass** mindestens eine Lichtquelle (4) und/oder mindestens ein Lichtsensor (5,6) kombiniert sind mit

- mindestens einer Optik (38,39) umfassend eine der folgenden Komponenten: Linse, Blende, Prisma, Beugungsgitter, optischer Spalt, Polarisator, optischer Filter, Lichtleiter und/oder
- einem elektronischen System zur Veränderung der Lichtquellenintensität (7) beziehungsweise der Lichtsensorsensitivität (8) und/oder
- mindestens einem Lichtsensor (24), der so positioniert und ausgerichtet ist, dass er nur Umgebungslicht, aber kein aus dem Reaktor (1) und/oder dem Reaktionsgemisch (2) austretendes Licht erfassen kann.

9. Vorrichtung nach einem der Ansprüche 7 bis 8 **dadurch gekennzeichnet, dass** die Vorrichtung

- mindestens ein Dosiersystem (12) und/oder mindestens eine Anschlussstelle für mindestens ein Dosiersystem (12) zur automatisierten Zugabe mindestens eines Stoffes zum Reaktionsgemisch (2) umfasst und/oder
- mindestens ein System und/oder mindestens eine Anschlussstelle für mindestens ein System zur Temperierung (25) des Reaktors (1) und/oder des Reaktionsgemisches (2) umfasst und/oder
- über Funk und/oder kabelgebunden mit mindestens einem Rechner (5) kommuniziert und/oder
- elektrische Energie aus mindestens einem Akkumulator (26) und/oder einer anderen elektrischen Energiequelle bezieht.

10. System zur Durchführung und Verarbeitung von Messungen der optischen Dichte (10) und/oder der Veränderung der optischen Dichte eines Reaktionsgemisches (2) in einem geschüttelten Reaktor (1), **dadurch gekennzeichnet, dass** das System mindestens eine Vorrichtung nach einem der Ansprüche 7 bis 9 umfasst, wobei das System ein Verfahren nach mindestens einem der Ansprüche 1 bis 6 verwendet.

## Claims

1. A method for determining the optical density and/or the change in optical density of a reaction mixture (2) in a shaken reactor (1),
   wherein

   - light from at least one light source (4) reaches the reaction mixture (2), and
   - the light leaving the reaction mixture (2) is detected by at least one light sensor (5, 6) so that both transmission through and diffusion at material situated in the light path are measured by different light source/ light sensor pairs, and
   - the reactor (1) and the reaction mixture (2) are shaken during the detection of the light by the at least one light sensor (5, 6), and
   - detection of the light by the at least one light sensor (5, 6) is performed at a frequency so that the shaking frequency is not an integer multiple of the detection frequency,
   - wherein no relative movement is taking place between the reactor and at least one light sensor (5, 6), and
   - for transmission and diffusion, several measurement points (20, 21) detected in a determined time interval by at least one light sensor (5, 6) are grouped into a measurement series (34),

   with each measurement series (34) being a curve varying depending on the shaking, the shape of which is caused by periodically variable levels of the reaction mixture in the respective light path, and wherein the measurement series (34) of transmission and diffusion are used for the determination of the optical densities and/or the change in optical density.

2. The method according to claim 1, **characterized in that**

   - detecting of the light by the at least one light sensor (5, 6) is performed at a frequency which is higher than the shaking frequency, and/or
   - at least one light path of at least one light source/ light sensor pair (4-5, 6) is not aligned in parallel with a shaking axis and/or a shaking plane.

3. The method according to any of claims 1 to 2, **characterized in that** from the series of measurements (34) detected in a determined time interval of transmission and diffusion, by means of at least one appropriate mathematical/ information technology method (36) and/or at least one model function, the optical density and/or the change in optical density and/or at least one fluid-mechanical parameter and/or at least a fluid-mechanical property of the reaction mixture (2) are determined in the measured time interval.

4. The method according to any of claims 1 to 3, **characterized in that** from at least one series of measurements (34) and/or at least one time series (35) of a parameter of the reaction mixture (2), determined from at least one measurement point (20, 21) and/or at least one series of measurements (34), at least one more parameter of the reaction mixture (2) is determined by means of at least one appropriate mathematical/ information technology method (36) and/or at least one model function.

5. The method according to any of claims 1 to 4, **characterized in that** at least one model function is regenerated and/or modified using at least one appropriate mathematical method (36) and/or at least one appropriate information technology algorithm (36).

6. The method according to any of claims 1 to 5, **characterized in that**

   - at least one dosing system (12) is controlled for adding at least one substance depending on the optical density and/or the change in optical density and/or at least one other process parameter, and/or
   - at least one more technical system affecting the process, in particular a system for temperature equalization (25) of the reaction mixture (2), is controlled depending on the optical density and/or the change in optical density and/or at least one more process parameter.

7. A device for performing the method according to any of claims 1 to 6, wherein

   - the device comprises at least one light source (4) which is positioned and aligned so that light emitted thereby may reach the reactor (1) under the conditions at least one type of shaking and directly and/or indirectly reach the reaction mixture (2), and
   - the device comprises at least one light sensor (5, 6), wherein at least one light sensor (5, 6) is positioned and aligned so that it may detect light emitted directly and/or indirectly from the reactor (1) and/or the reaction mixture (2) so that both transmission through and diffusion at the substance situated in the light path can be measured by different light source/ light sensor pairs, and
   - the device comprises shaking equipment, and
   - the device comprises at least one computer (9, 10), wherein the computer (9, 10) is implemented for at least one of the following purposes: recording the measurement values, storing the measurement values, processing the measurement values, representing the measurement values and/or processing results,

   wherein the device is configured so that:

   - the reactor (1) and the reaction mixture (2) are shaken while the light is being detected by the at least one light sensor (5, 6),
   - detection of the light by the at least one light sensor (5, 6) is performed at a frequency so that the shaking frequency is not an integer multiple of the detection frequency,
   - no relative movement is taking place between the reactor and the at least one light sensor (5, 6),
   - for transmission and diffusion, respectively several measurement points (20, 21) detected in a determined time interval by the at least one light sensor (5, 6) are grouped into a series of measurements (34), each series of measurements being a curve varying due to the shaking, the shape of which is caused by periodically varying levels of the reaction mixture in the respective light path, and
   - the series of measurements (34) of transmission and diffusion are used for the determination of the optical densities and/or the change in optical density.

8. The device according to claim 7, **characterized in that** at least one light source (4) and/or at least one light sensor (5, 6) are combined with

   - at least one optical system (38, 39) comprising one of the following components: a lens, an aperture, a prism, a diffraction grating, an optical slit, a polarizer, an optical filter, an optical waveguide, and/or
   - an electronic system for changing light source intensity (7) or light sensor sensitivity (8), and/or
   - at least one light sensor (24) which is positioned and aligned so that it can only detect ambient light but not light leaving the reactor (1) and/or the reaction mixture (2).

**9.** The device according to any of claims 7 to 8, **characterized in that** the device

- comprises at least one dosing system (12) and/or at least one connecting point for at least one dosing system (12) for the automated addition of at least one substance to the reaction mixture (2), and/or
- comprises at least one system and/or at least one connecting point for at least one system for temperature equalization (25) of the reactor (1) and/or the reaction mixture (2), and/or
- communicates via radio and/or wire with at least one computer (5), and/or
- receives electrical energy from at least one accumulator (26) and/or one other electrical energy source.

**10.** A system for performing and processing measurements of the optical density (10) and/or the change in optical density of a reaction mixture (2) in a shaken reactor (1), **characterized in that** the system comprises at least one device according to any of claims 7 to 9, the system using a method according to at least one of claims 1 to 6.

**Revendications**

**1.** Procédé permettant de déterminer la densité optique et/ou la modification de la densité optique d'un mélange réactionnel (2) dans un réacteur agité (1),
dans lequel

- la lumière d'au moins une source de lumière (4) atteint le mélange réactionnel (2), et
- la lumière sortant du mélange réactionnel (2) est détectée par au moins un capteur de lumière (5, 6) de sorte qu'à la fois une transmission à travers et une diffusion au niveau d'une matière se trouvant sur le chemin optique sont mesurées par différentes paires de source de lumière/ capteur de lumière, et
- le réacteur (1) et le mélange réactionnel (2) sont agités pendant la détection de la lumière par ledit au moins un capteur de lumière (5, 6), et
- la détection de la lumière par ledit au moins un capteur de lumière (5, 6) est effectuée à une fréquence telle que la fréquence d'agitation n'est pas un multiple entier de la fréquence de détection,
- dans lequel aucun mouvement relatif n'a lieu entre le réacteur et ledit au moins un capteur de lumière (5, 6), et
- plusieurs points de mesure (20, 21) détectés dans un intervalle de temps déterminé par au moins un capteur de lumière (5, 6) sont regroupés en une série de mesures (34) pour la transmission et pour la diffusion respectivement,

dans lequel la série de mesures est une courbe variable en fonction de l'agitation dont la forme est provoquée par des hauteurs, variant périodiquement, du mélange réactionnel sur le chemin optique respectif, et dans lequel les séries de mesure (34) de la transmission et de la diffusion sont utilisées pour déterminer les densités optiques et/ou la modification de la densité optique.

**2.** Procédé selon la revendication 1, **caractérisé en ce que**

- la détection de la lumière par ledit au moins un capteur de lumière (5, 6) est effectuée à une fréquence qui est supérieure à la fréquence d'agitation, et/ou
- au moins un chemin optique d'au moins une paire de source de lumière/ capteur de lumière (4-5, 6) n'est pas aligné en parallèle à un axe d'agitation et/ou à un plan d'agitation.

**3.** Procédé selon l'une quelconque des revendications 1 à 2, **caractérisé en ce qu'**à partir des séries de mesures (34) détectées dans un intervalle de temps déterminé de la transmission et de la diffusion, la densité optique et/ou la modification de la densité optique et/ou au moins un paramètre fluidique-mécanique et/ou au moins une propriété fluidique-mécanique du mélange réactionnel (2) sont déterminés dans l'intervalle de temps mesuré à l'aide d'au moins un procédé mathématique/informatique adapté (36) et/ou d'au moins une fonction modèle.

**4.** Procédé selon l'une quelconque des revendications 1 à 3, **caractérisé en ce qu'**à partir d'au moins une série de mesures (34) et/ou d'au moins une série chronologique (35) d'un paramètre du mélange réactionnel (2) déterminé à partir d'au moins un point de mesure (20, 21) et/ou d'au moins une série de mesures (34), au moins un autre paramètre du mélange réactionnel (2) est déterminé à l'aide d'au moins un procédé mathématique/informatique adapté (36) et/ou d'au moins une fonction modèle.

**5.** Procédé selon l'une quelconque des revendications 1 à 4, **caractérisé en ce qu'**au moins une fonction modèle est régénérée et/ou modifiée en utilisant au moins un procédé mathématique adapté (36) et/ou au moins un algorithme informatique adapté (36).

6. Procédé selon l'une quelconque des revendications 1 à 5, **caractérisé en ce que**

- au moins un système de dosage (12) est commandé pour ajouter au moins une substance en fonction de la densité optique et/ou de la modification de la densité optique et/ou d'au moins un autre paramètre de processus, et/ou
- au moins un autre système technique affectant le processus, en particulier un système pour l'équilibrage de température (25) du mélange réactionnel (2), est commandé en fonction de la densité optique et/ou de la modification de la densité optique et/ou d'au moins un autre paramètre de processus.

7. Dispositif permettant d'exécuter le procédé selon l'une quelconque des revendications 1 à 6, dans lequel

- le dispositif comprend au moins une source de lumière (4) qui est positionnée et alignée de telle sorte que la lumière qu'elle émet atteint le réacteur (1) sous les conditions d'au moins un type d'agitation et peut atteindre le mélange réactionnel (2) directement et/ou indirectement, et
- le dispositif comprend au moins un capteur de lumière (5, 6), dans lequel au moins un capteur de lumière (5, 6) est positionné et aligné de façon à pouvoir détecter la lumière émise directement et/ou indirectement par le réacteur (1) et/ou le mélange réactionnel (2) de sorte qu'il est possible de mesurer par différentes paires de source de lumière/ capteur de lumière à la fois une transmission à travers et une diffusion au niveau de la matière se trouvant sur le chemin optique, et
- le dispositif comprend un moyen d'agitation, et
- le dispositif comprend au moins un ordinateur (9, 10), dans lequel ledit au moins un ordinateur (9, 10) est mis en oeuvre pour au moins l'un des usages suivants : l'enregistrement des valeurs de mesure, le stockage des valeurs de mesure, le traitement des valeurs de mesure, la représentation des valeurs de mesure et/ou des résultats de traitement,

dans lequel le dispositif est configuré de telle sorte que :

- le réacteur (1) et le mélange réactionnel (2) sont agités pendant la détection de la lumière par ledit au moins un capteur de lumière (5, 6),
- la détection de la lumière par ledit au moins un capteur de lumière (5, 6) est effectuée à une fréquence telle que la fréquence d'agitation n'est pas un multiple entier de la fréquence de détection,
- aucun mouvement relatif n'a lieu entre le réacteur et ledit au moins un capteur de lumière (5, 6),
- plusieurs points de mesure (20, 21) détectés dans un intervalle de temps déterminé par ledit au moins un capteur de lumière (5, 6) sont regroupés en une série de mesures (34) pour la transmission et pour la diffusion respectivement, chaque série de mesures est une courbe variable en fonction de l'agitation dont la forme est provoquée par des hauteurs, variant périodiquement, du mélange réactionnel sur le chemin optique respectif, et
- les séries de mesure (34) de la transmission et de la diffusion sont utilisées pour déterminer les densités optiques et/ou la modification de la densité optique.

8. Dispositif selon la revendication 7, **caractérisé en ce qu'**au moins une source de lumière (4) et/ou au moins un capteur de lumière (5, 6) sont combinés avec

- au moins une optique (38, 39) comprenant l'un des composants suivants : une lentille, un diaphragme, un prisme, un réseau de diffraction, une fente optique, un polariseur, un filtre optique, un guide de lumière, et/ou
- un système électronique pour modifier l'intensité de source de lumière (7) ou la sensibilité de capteur de lumière (8), et/ou
- au moins un capteur de lumière (24) qui est positionné et aligné de telle sorte qu'il ne peut détecter que la lumière ambiante mais pas la lumière sortant du réacteur (1) et/ou du mélange réactionnel (2).

9. Dispositif selon l'une quelconque des revendications 7 à 8, **caractérisé en ce que** le dispositif

- comprend au moins un système de dosage (12) et/ou au moins un point de raccordement pour au moins un système de dosage (12) pour ajouter de manière automatisée au moins une substance au mélange réactionnel (2), et/ou
- comprend au moins un système et/ou au moins un point de raccordement pour au moins un système pour l'équilibrage de température (25) du réacteur (1) et/ou du mélange réactionnel (2), et/ou
- communique par radio et/ou câble avec au moins un ordinateur (5), et/ou
- reçoit de l'énergie électrique d'au moins un accumulateur (26) et/ou d'une autre source d'énergie électrique.

**10.** Système permettant d'effectuer et de traiter des mesures de la densité optique (10) et/ou de la modification de la densité optique d'un mélange réactionnel (2) dans un réacteur agité (1), **caractérisé en ce que** le système comprend au moins un dispositif selon l'une quelconque des revendications 7 à 9, le système utilisant un procédé selon au moins l'une des revendications 1 à 6.

Abb. 1

Abb. 2

# Abb. 3

EP 3 100 026 B1

**Abb. 4**

**Abb. 5**

EP 3 100 026 B1

# Abb. 6

Abb. 7

## Abb. 9

- ● ● ● elektronische Verbindung/Kommunikation zwischen Komponenten der Abbildung
- elektronische Verbindung/Kommunikation mit nicht abgebildeter Komponente
- ⬆ Lichtstrahl/Lichtpfad
- Lichtsensor **5/6/24**
- Auswertungs- und Steuereinheit **8** für mindestens einen Lichtsensor **5/6/24**
- Lichtquelle **4**
- Steuereinheit **7** für mindestens eine Lichtquelle **4**
- Temperierungssystem **25**
- Dosiersystem **12**
- Rechner **9**
- Zuzugebende Komponente **14**
- Reaktionsgemisch **2** (im Schnitt)
- Reaktionsgemisch **2** (kein Schnitt)

## Abb. 8

Abb. 10

# Abb. 11

◇ Transmissionssignal (20) bei 0,33 g/L Biomassekonzentration    ◇ Streusignal (21) bei 0,33 g/L Biomassekonzentration

◇ Transmissionssignal (20) bei 0,99 g/L Biomassekonzentration    ◇ Streusignal (21) bei 0,99 g/L Biomassekonzentration

◇ Transmissionssignal (20) bei 2,04 g/L Biomassekonzentration    ◇ Streusignal (21) bei 2,04 g/L Biomassekonzentration

Abb. 12

Abb. 13

$R^2 = 0,990$

$R^2 = 0,997$

Biomassekonzentration [g/L]

$\log_{10}$ (Faltungsmittelwertquotient) [-]

◆ von Transmissionsmessreihen (34/20), mit Umgebungslicht, normalisiert

■ von Transmissionsmessreihen (34/20), ohne Umgebungslicht, normalisiert

—— Linear (von Transmissionsmessreihen (34/20), mit Umgebungslicht, normalisiert)

----- Linear (von Transmissionsmessreihen (34/20), ohne Umgebungslicht, normalisiert)

# Abb. 14

Legend:
- ◆ von Transmissionsmessreihen (34/20), ohne Umgebungslicht, ungebaffelt, normalisiert
- ▲ von Transmissionsmessreihen (34/20), ohne Umgebungslicht, gebaffelt, normalisiert
- ■ von Transmissionsmessreihen (34/20), mit Umgebungslicht, ungebaffelt, normalisiert
- —— Linear (von Transmissionsmessreihen (34/20), ohne Umgebungslicht, ungebaffelt, normalisiert)
- — · — Linear (von Transmissionsmessreihen (34/20), ohne Umgebungslicht, gebaffelt, normalisiert)
- ------ Linear (von Transmissionsmessreihen (34/20), mit Umgebungslicht, ungebaffelt, normalisiert)

EP 3 100 026 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20090075248 A1
- US 8405033 B2
- JP 02012217426 A
- US 6673532 B2
- US 7041493 B2
- DE 102004017039 A1